# EUROPEAN PATENT SPECIFICATION

(11) **EP 1 646 910 B1**
(45) Date of publication and mention of the grant of the patent: **19.08.2015**
(21) Application number: 04756940.5
(22) Date of filing: 14.07.2004
(51) Int. Cl.: A61M 16/06

(54) **NASAL INTERFACE APPARATUS**
NASENSCHNITTSTELLENVORRICHTUNG
DISPOSITIF D'INTERFACE NASAL

(30) Priority: 17.07.2003 US 488939 P; 02.03.2004 US 549606 P; 13.05.2004 US 570755 P
(43) Date of publication of application: 19.04.2006
(73) Proprietor: CareFusion 202, Inc., San Diego CA 92130 (US)
(72) Inventor: THOMLINSON, Marguerite, Irvine, California 92620 (US); CHU, Edmond, San Diego, California 92131 (US); VENTRESS, Jim, Tustin, California 92705 (US)
(74) Representative: Richards, John
(86) International application number: PCT/US2004/022470
(87) International publication number: WO 2005/010608

(56) References cited:
- US-A- 4 782 832
- US-A- 5 065 756
- US-A- 5 595 174
- US-A- 5 595 174
- US-A1- 2003 079 749
- US-A1- 2003 116 163
- US-B1- 6 431 172
- US-B1- 6 431 172
- US-B2- 6 679 265
- US-B2- 6 863 069

## Description

### RELATED APPLICATIONS

This application claims priority to U.S. provisional patent application Serial Nos. 60/488,939 filed on July 17, 2003, 60/549,606 filed on March 2, 2004, and 60/570,755 filed on May 13, 2004. The above-identified applications are incorporated by reference as if set forth fully herein.

### FIELD OF THE INVENTION

The present invention relates generally to devices used to delivery positive airway pressure to a patient for the treatment of sleep apnea. More specifically, the present invention relates to nasal interface devices used to deliver positive airway pressure to a patient for the treatment of sleep apnea.

### BACKGROUND

Sleep apnea is a potentially lethal affliction in which breathing stops recurrently during sleep. Sleep apnea may be of the obstructive type (sometimes known as the pickwickian syndrome) in which the upper airway is blocked in spite of airflow drive; the central type with decreased respiratory drive; or a mixed type. Breathing may cease for periods long enough to cause or to exacerbate cardiac conditions, and may be accompanied by swallowing of the tongue. Sleep apnea frequently results in fitful periods of both day and night sleeping with drowsiness and exhaustion, leaving the patient physically and mentally debilitated.

In recent years it has been found that various forms of positive airway pressure during sleep can be an effective form of therapy for the apnea sufferer. Ventilation can be applied in the form of Continuous Positive Airway Pressure (CPAP) in which a positive pressure is maintained in the airway throughout the respiratory cycle, Bilevel Positive Airway Pressure (BIPAP) in which positive pressure is maintained during inspiration but reduced during expiration, and Intermittent Mechanical Positive Pressure Ventilation in which pressure is applied when an episode of apnea is sensed. Positive airway pressure devices have traditionally employed either a face mask which only covers the patient's nose, or nasal interface between the ventilation device and the patient's airway.

Examples of prior art devices are given in US 4 782 832 A, US 6 431 172 B1, US 2003/079749 A1, US 2003/116163 A1, US 5 595 174 A and US 5 065 756 A.

### SUMMARY OF THE INVENTION

In the invention, a nasal interface as claimed is disclosed.

A method of providing ventilation for an individual is also disclosed. In one embodiment, this method includes providing a nasal interface with a pair of nasal prongs, where each prong has a bore and at least one flap at or near a tip of the prong, a body having a distal portion and a proximal portion, where the distal portion and proximal portions form a chamber when assembled. In one embodiment, the method includes a proximal portion having apertures to receive second ends of the prongs, and wherein the nasal prongs releasably engage with the proximal portion, and the chamber or body communicates with the bores. In one embodiment, the chamber includes at least one exhalation port within the distal portion of the body for directing exhaled gas away from the chamber, and at least one gas inlet on the distal portion communicating with the chamber. In one embodiment, the method includes operatively connecting the nasal interface with a ventilation apparatus; and placing the nasal interface in sealing contact using flaps with nares of the individual.

The further embodiments of the invention are restricted to the ones claimed in the depending claims. Where in the following the word invention is used and/or features are presented as optional this should be interpreted in such a way that protection is sought for the invention as claimed.

### BRIEF DESCRIPTION OF THE DRAWINGS

Fig. 1 is a bottom view of an embodiment of a nasal interface of the invention.
Fig. 2 is a side view of an embodiment of the nasal interface of Fig. 1.
Fig. 3 is a back view of an embodiment of the nasal interface of Fig. 1.
Fig. 4 is a front view of an embodiment of the nasal interface of Fig. 1.
Fig. 5 is a view of an embodiment of the nasal interface of Fig. 1 in use.
Fig. 6 is a top view of another embodiment of a nasal interface of the invention.
Fig. 7 is a perspective view of the nasal interface of Fig. 6.
Fig. 8 is a perspective view of another embodiment of a nasal interface of the invention.
Fig. 9 is an exploded view of the nasal interface of Fig. 1.
Fig. 10A is a perspective view of an embodiment of a nasal interface of the invention.
Fig. 10B is an exploded view of the nasal interface of Fig. 10A.
Fig. 11 is an exploded view of the nasal interface of Fig. 8.
Fig. 12A is a perspective view of the proximal portion of Fig. 8.
Fig. 12B is a top view of the proximal portion of Fig. 8;
Fig. 12C is a front view of the proximal portion of Fig. 8.
Fig. 12D is a side view of the proximal portion of Fig. 8.
Fig. 13A is a front perspective view of the proximal portion of Fig. 10B.
Fig. 13B is a top view of the proximal portion of Fig. 10B.
Fig. 13C is an enlarged view of proximal ridge of the proximal portion of Fig. 13F.
Fig. 13D is a rear perspective view of the proximale portion of Fig. 10B.
Fig. 13E is a front view of the proximal portion of Fig. 10B.
Fig. 13F is a cross sectional view of the proximal portion of Fig. 13E along line B-B.
Fig. 13G is a back view of the proximal portion of Fig. 10B.
Fig. 13H is a cross sectional view of the proximal portion of Fig. 13E along line A-A.
Fig. 13I is an enlarged view of an opening in the proximal portion of Fig. 13E.
Fig. 14A is a top view of the distal portion of Fig. 8.
Fig. 14B is a perspective view of the distal portion of Fig. 8.
Fig. 14C is a front view of the distal portion of Fig. 8.
Fig. 14D is a side view of the distal portion of Fig. 8.
Fig. 15A is a front perspective view of a distal portion of Fig. 26.
Fig. 15B is rear perspective view of the distal portion of Fig. 15A.
Fig. 15C is a top view of the distal portion of Fig. 15A.
Fig. 15D is a front view of the distal portion of Fig. 15A.
Fig. 15E is a side view of the distal portion of Fig. 15A.
Fig. 15F is a back view of the distal portion of Fig. 15A.
Fig. 15G is a cross sectional view of the distal portion of Fig. 15E alone line A-A.
Fig. 16A is a top view of the distal portion of Fig. 15A.
Fig. 16B is an enlarged view of the exhalation ports of the distal portion of Fig. 16C.
Fig. 16C is a front view of the distal portion of Fig. 16A.
Fig. 16D is a cross sectional view of the distal portion of Fig. 16C along line B-B.
Fig. 16E is a back view of the distal portion of Fig. 16A.
Fig. 16F is a cross sectional view of the distal portion of Fig. 16C along line A-A.
Fig. 16G is a perspective view of the distal portion of Fig. 16A.
Fig. 16H is an enlarged cross sectional view of an exhalation port of the distal portion of Fig. 16F.
Fig. 17A is a front perspective view of the distal portion of Fig. 10B.
Fig. 17B is a rear perspective view of the distal portion of Fig. 10B.
Fig. 17C is a top view of the distal portion of Fig. 10B.
Fig. 17D is an enlarged view of a lip on the distal portion of Fig. 17C.
Fig. 17E is a front view of the distal portion of Fig. 10B.
Fig. 17F is a cross sectional view of the distal portion of Fig. 17E along line A-A.
Fig. 17G is a cross sectional view of the distal portion of Fig. 17E along line B-B.
Fig. 17H is a back view of the distal portion of Fig. 10B.
Fig. 18A is a perspective view of one embodiment of a nasal prong of the invention.
Fig. 18B is a side view of the nasal prong of Fig. 18A.
Fig. 18C is a bottom view of the nasal prong of Fig. 18A.
Fig. 18D is a view of the nasal prong of Fig. 18B rotated 90 degrees around its longitudinal axis.
Fig. 18E is a top view of the nasal prong of Fig. 18A.
Fig. 19A is a perspective view of one embodiment of dual nasal prongs of the invention.
Fig. 19B is a side view of the dual nasal prongs of Fig. 19A.
Fig. 19C is a cross sectional view of one of the prongs of Fig. 19B along line B-B.
Fig. 19D is a top view of the dual nasal prongs of Fig. 19A.
Fig. 19E is a side view of one nasal prong of Fig. 19A.
Fig. 19F is a bottom view of the dual nasal prongs of Fig. 19A.
Fig. 19G is a cross sectional view of the prongs of Fig. 19D along line A-A.
Fig. 19H is an enlarged view of tip of a nasal prong of Fig. 19G.
Fig. 20A is a front perspective view of one embodiment of dual nasal prongs of the invention.
Fig. 20B is a rear perspective view of the dual nasal prongs of Fig. 20A.
Fig. 20C is a side view of the dual nasal prongs of Fig. 20A.
Fig. 20D is a cross sectional view of one of the prongs of Fig. 20C along line B-B.
Fig. 20E is a top view of the dual nasal prongs of Fig. 20A.
Fig. 20F is a side view of one nasal prong of Fig. 20A;
Fig. 20G is a bottom view of the dual nasal prongs of Fig. 20A.
Fig. 20H is a cross sectional view of the prongs of Fig. 20E along line A-A.
Fig. 20I is an enlarged view of the tip of the nasal prong of Fig. 20H.
Fig. 21A is a front perspective view of one embodiment of dual nasal prongs of the invention.
Fig. 21B is a rear perspective view of the dual nasal prongs of Fig. 21A.
Fig. 21C is a side view of the dual nasal prongs of Fig. 21A.
Fig. 21D is a cross sectional view of one of the prongs of Fig. 21C along line B-B.
Fig. 21E is a top view of the dual nasal prongs of Fig. 21A.
Fig. 21F is a side view of one nasal prong of Fig. 21A.
Fig. 21G is a bottom view of the dual nasal prongs of Fig. 21A.
Fig. 21H is a cross sectional view of the prongs of Fig. 21E along line A-A.
Fig. 21I is an enlarged view of the tip of a nasal prong of Fig. 21H.
Fig. 22A is a front perspective view of one embodiment of dual nasal prongs of the invention.
Fig. 22B is a rear perspective view of the dual nasal prongs of Fig. 22A.
Fig. 22C is a side view of the dual nasal prongs of Fig. 22A.
Fig. 22D is a cross sectional view of one of the prongs of Fig. 22C along line B-B.
Fig. 22E is a top view of the dual nasal prongs of Fig. 22A.
Fig. 22F is a side view of one nasal prong of Fig. 22A.
Fig. 22G is a bottom view of the dual nasal prongs of Fig. 22A.
Fig. 22H is a cross sectional view of the prongs of Fig. 22E along line A-A.
Fig. 22I is an enlarged view of the tip of a nasal prong of Fig. 22H.
Fig. 23A is a front perspective view of one embodiment of dual nasal prongs of the invention.
Fig. 23B is a rear perspective view of the dual nasal prongs of Fig. 23A.
Fig. 23C is a side view of the dual nasal prongs of Fig. 23A.
Fig. 23D is a cross sectional view of one of the prongs of Fig. 23C along line B-B.
Fig. 23E is a top view of the dual nasal prongs of Fig. 23A.
Fig. 23F is a side view of one nasal prong of Fig. 23A.
Fig. 23G is a bottom view of the dual nasal prongs of Fig. 23A.
Fig. 23H is a cross sectional view of the prongs of Fig. 23E along line A-A.
Fig. 23I is an enlarged view of the tip of a nasal prong of Fig. 23H.
Fig. 24A is a front perspective view of one embodiment of dual nasal prongs of the invention.
Fig. 24B is a side view of the dual nasal prongs of Fig. 24A.
Fig. 24C is a side view of one nasal prong of Fig. 24A.
Fig. 24D is a top view of the dual nasal prongs of Fig. 24A.
Fig. 24E is a side view of one nasal prong of Fig. 24A.
Fig. 24F is a bottom view of the dual nasal prongs of Fig. 24A.
Fig. 24G is a cross sectional view of the prongs of Fig. 24D along line A-A.
Fig. 24H is an enlarged view of the tip of a nasal prong of Fig. 24G.
Fig. 25A is a perspective view of an exhalation port of the invention.
Fig. 25B is a top view of the exhalation port of Fig. 25A.
Fig. 25C is a front view of the exhalation port of Fig. 25A.
Fig. 25D is a cross sectional view of the exhalation port of Fig. 25C along line A-A.
Fig. 25E is a bottom view of the exhalation port of Fig. 25A.
Fig. 26 is an exploded view of one embodiment of a system of the invention.
Fig. 27 is an assembled view of the system shown in Fig. 26.
Fig. 28A is a perspective view of one embodiment of a strap attachment plate of the invention.
Fig. 28B is a top view of the strap attachment plate of Fig. 28A.
Fig. 28C is a side view of the strap attachment plate of Fig. 28A.
Fig. 28D is a front view of the strap attachment plate of Fig. 28A.
Fig. 28E is a second front view of the strap attachment plate of Fig. 28A.
Fig. 29 is an assembled view of one embodiment of a system of the invention.
Fig. 30 is an assembled view of one embodiment of a system of the invention.
Fig. 31 is one embodiment of a system of the invention in use.
Fig. 32 is one embodiment of a system-of the invention.
Fig 33A is a perspective view of one embodiment a tubing connector of the invention.
Fig 33B. is a top view of the tubing connector of Fig. 33A.
Fig 33C is a side view of the tubing connector of Fig. 33A.
Fig 33D is a bottom view of the tubing connector of Fig. 33A.
Fig. 34 is one embodiment of a strap system of the invention.
Fig. 35 is an exploded view of one embodiment of a system of the invention.
Fig. 36 is an exploded view of one embodiment of a system of the invention.
Fig. 37 is an assembled view of the system of Fig. 35.
Fig. 38 is an assembled view of the system of Fig. 36.
Fig. 39A is a top assembled view of one embodiment of a nasal interface and tubing of the invention.
Fig. 39B is a side assembled view of one embodiment of a nasal interface and tubing of the invention.
Fig. 39C is a front view of one embodiment of a nasal interface of the invention.
Fig. 40A is a perspective view of one embodiment of a sealing ring of the invention.
Fig. 40B is another perspective view of one embodiment of a sealing ring of the invention.
Fig. 40C is a top view of one embodiment of a sealing ring of the invention.
Fig. 40D is a front view of one embodiment of a sealing ring of the invention.
Fig. 40E is a side view of one embodiment of a sealing ring of the invention.
Fig. 40F is a back view of one embodiment of a sealing ring of the invention.
Fig. 40G is a cross sectional view of the sealing ring of Fig. 40F along line B-B.
Fig. 41A is a top view of one embodiment of a sealing ring of the invention.
Fig. 41B is a side view of one embodiment of a sealing ring of the invention along the line D-D in Fig. 41A.
Fig. 41C is a cross sectional view of the sealing ring of Fig. 41D along line B-B.
Fig. 41D is a front view of one embodiment of a sealing ring of the invention.
Fig. 41E is a side view of one embodiment of a sealing ring of the invention.
Fig. 41F is a back view of one embodiment of a sealing ring of the invention.
Fig. 41G is a cross sectional view of the sealing ring of Fig. 41D along line A-A.
Fig. 41H is a perspective view of one embodiment of a sealing ring of the invention.
Fig. 42A is a top view of one embodiment of a loop of the invention.
Fig. 42B is a side view of one embodiment of a strap of the invention.
Fig. 42C is a side view of one embodiment of a strap of the invention.
Fig. 42D is a perspective view of one embodiment of a strap system of the invention.
Fig. 42E is a side view of one embodiment of a strap of the invention.
Fig. 42F is a top view of one embodiment of a strap and loop of the invention.
Fig. 43 is an exploded view of one embodiment of a width-expanding nasal interface of the invention.
Fig. 44 is an exploded view of one embodiment of a width-expanding nasal interface of the invention.
Fig. 45 is an exploded view of one embodiment of a width-expanding nasal interface of the invention.
Fig. 46 is an exploded view of one embodiment of a width-expanding nasal interface of the invention.
Fig. 47 is an exploded view of one embodiment of a width-expanding nasal interface of the invention.
Fig. 48 is an exploded view of one embodiment of a width-expanding nasal interface of the invention.
Fig. 49 is an assembled view of one embodiment of a width-expanding nasal interface of the invention.
Fig. 50 is one embodiment of a pair of nasal inserts of the present invention.
Fig. 51A is a top view of one embodiment of a pair of nasal inserts of the present invention.
Fig. 51B is a side view of one embodiment of a pair of nasal inserts of the present invention.
Fig. 51C is a perspective view of one embodiment of a pair of nasal inserts of the present invention.
Fig. 52 is a top view of one embodiment of a nasal interface of the present invention.
Fig. 53 is a top view of one embodiment of a nasal interface of the present invention.
Fig. 54A is a top view of one embodiment of a nasal interface of the present invention.
Fig. 54B is a side view of one embodiment of a nasal interface of the present invention.
Fig. 55 is an exploded view of one embodiment of a nasal interface of the present invention.
Fig. 56 is an assembled view of one embodiment of a nasal interface of the present invention.
Fig. 57 is an exploded view of one embodiment of a nasal interface of the present invention.
Fig. 58 is an assembled view of one embodiment of a nasal interface of the present invention.
Fig. 59 is an exploded view of one embodiment of a nasal interface of the present invention.
Fig. 60 is an assembled view of one embodiment of a nasal interface of the present invention.

### DETAILED DESCRIPTION OF THE PREFERRED EMBODIMENTS

The present invention is directed to nasal interfaces, nasal interface components, systems including nasal interfaces and methods of use.

As used herein, the terms "proximal portion" and "distal portion" refer to the components of the invention that form the body of the nasal interface. Specifically, the component of the invention that is closest to the user and typically contains openings for one or more nasal prongs is referred to as the "proximal portion" and the component that is furthest from the user and typically includes at least one gas inlet, is referred to as the "distal portion."

Nasal interfaces, nasal interface components, nasal interface systems and methods of use are described in detail hereafter.

### I. Nasal Interfaces

### A. Assembled Nasal Interfaces / Bodies

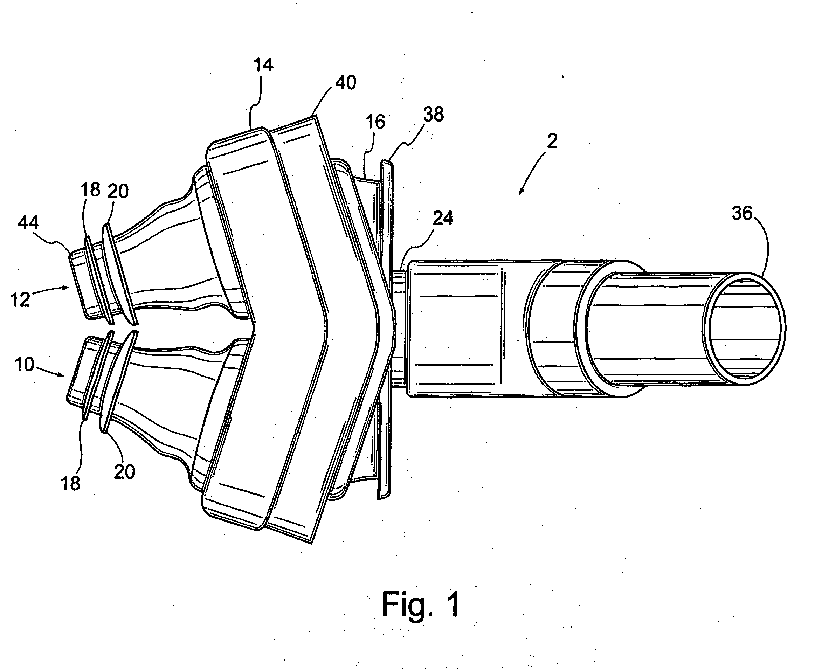

As noted briefly above, the invention provides nasal interfaces suitable for ventilation applications, such as continuous positive airway pressure (CPAP) applications, and bi-level positive airway pressure (BIPAP) applications, and intermittent (non-continuous) positive pressure (IPPB) applications. Figs. 1 through 8 show views of assembled nasal interfaces of embodiments of the present invention.

The individual components of the nasal interface, including the nasal prongs 10 and 12, distal portion 16 and proximal portion 14, can be assembled to form a nasal interface (also referred to herein as "a nasal interface body" or "interface body") of the present invention. In preferred embodiments, the nasal interface components of the invention can typically be assembled or disassembled without the use of any tools or fasteners or adhesives. In some embodiments, fasteners and/or adhesives may be used to assemble a nasal interface if a more permanent assembly is desired.

As described further below, in some embodiments, the nasal interface accommodates and holds adjacent elements in a removably or releasably locking or sealing engagement. For example, to form a nasal interface according to one embodiment of the present invention, proximal portion 14 can slide onto the distal portion 16 to form a nasal interface that is held together in a friction fit and/or mechanical fit, as shown in the figures. The assembled nasal interface may be of any shape, including cylindrical, rectangular, or any other regular or irregular shape.

The ease of assembly and disassembly also facilitates cleaning of the nasal interface. Once disassembled, the nasal interface of the invention can be quickly air dried after immersion in cleaning solution (e.g. soap and water). Nasal interfaces of the invention have a minimal amount of crevasses where bacteria and moisture can hide and grow, thereby reducing the incidence of possible infection. Another advantage of the multi-components nasal interface is that the interface can be much more easily manufactured and the cost of manufacturing will be much lower. Each piece may be manufactured separately in a mold by, for example, injection molding, cast molding and any other molding process.

Proximal portion 14 and distal portion 16, when assembled together, create a relatively small chamber. A small chamber translates to a smaller volume for dead space, thus minimizing the accumulation of carbon dioxide.

The typical volume of an assembled embodiment of the invention can be from about 20 ml. to about 200 ml. In some embodiments, the volume of a device including proximal portion 14, distal portion 16 and nasal prongs 10 and 1.2 can be less than 1ml. In one embodiment, the volume of an entire device, including a body having a proximal portion 14, distal portion 16, nasal prongs 10 and 12, and tubing 90 and any additional optional components, can be about 100 ml. It should be noted that the volume of embodiments of the invention should be minimized to reduce or eliminate dead space. The typical flow rate of gas through an assembled embodiment can be about 5 to 120 liters of gas per minute, preferably about 5 to about 60 liters of gas per minute.

In some embodiments of the invention, the nasal interface can be connected to ventilator apparatus via additional tubing or the like. The ventilator apparatus can be used to provide air, enriched air (22% to 100% Oxygen), or alternative gas mixtures to a patient in need thereof.

### B. Assembled Nasal Interface Embodiments

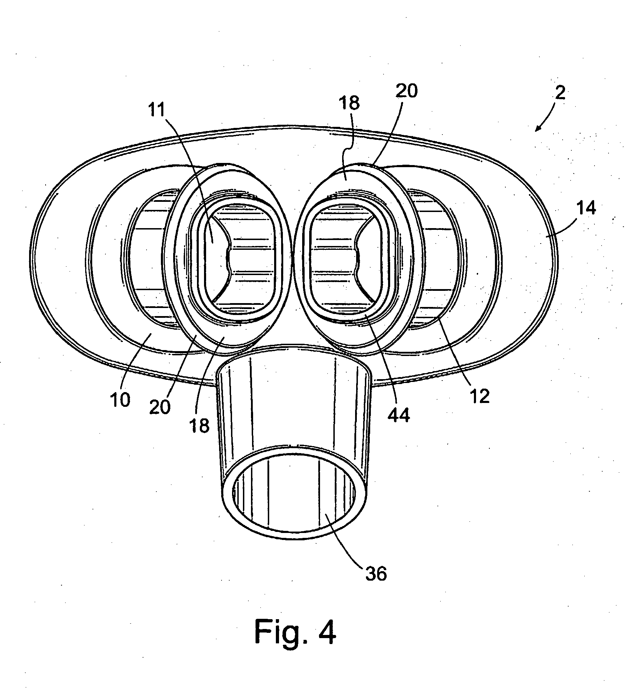

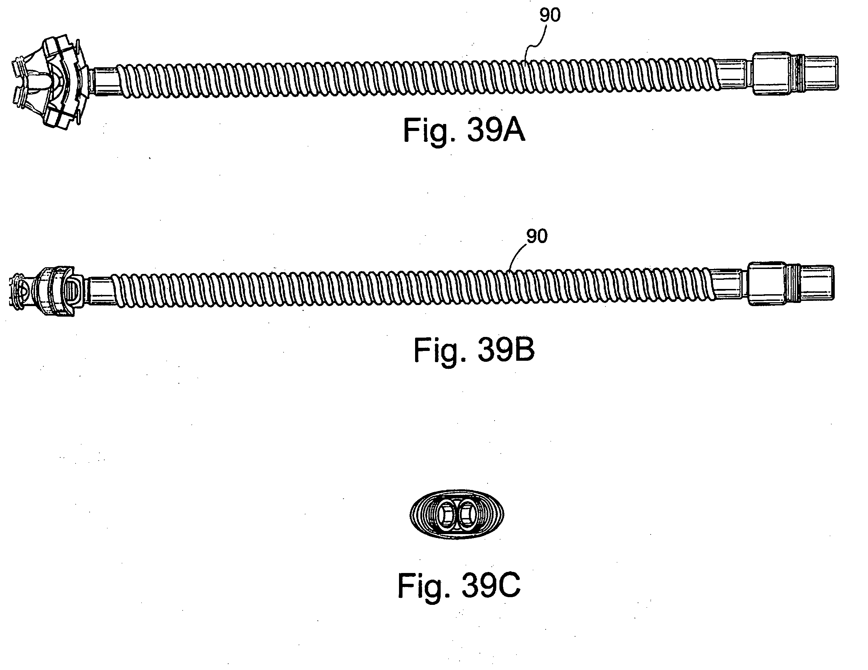

Figs. 1 through 5 provide views of one embodiment of the present invention, including a nasal interface body having a first nasal prong 10 and a second nasal prong 12. Each nasal prong has a bore, referred to singly as bore 11, shown for example in Fig. 4. In certain embodiments, one or both nasal prongs of the invention includes a first sealing portion (e.g., first flap 18) and one or more optional additional sealing portions (e.g., optional additional flap 20). Fig. 39C also shows a front view of one embodiment of the present invention.

The embodiment depicted in Figs. 1 through 5 includes a proximal portion 14 and a distal portion 16, which combine to form a nasal interface body. One embodiment can further include a sealing ring 40, which can lock together and maintains an airtight seal between proximal portion 14 and distal portion 16. Sealing ring 40 is described further below.

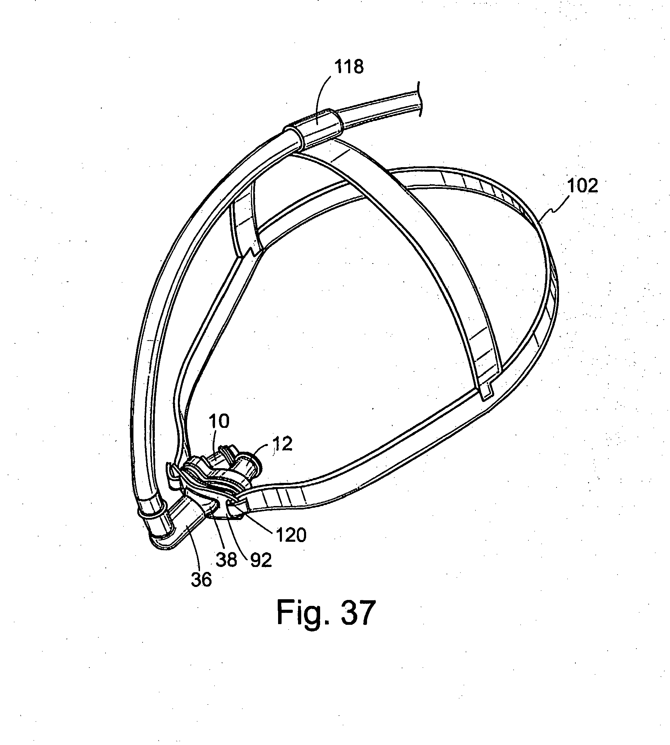

A nasal interface body 2 according to the present invention can also include one or more locking tabs 38 on the distal portion 16. The locking tabs 38 can be used to releasably engage a strap attachment plate 92, as depicted in Fig. 37 and described further below.

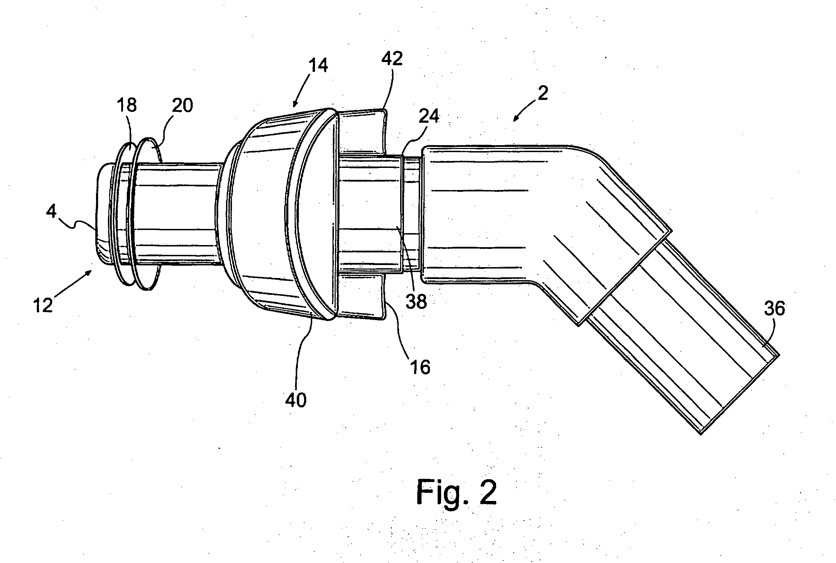

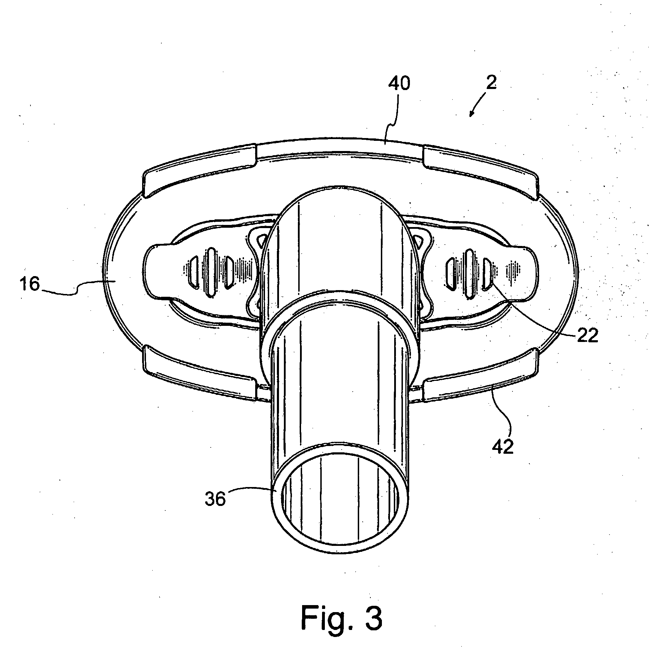

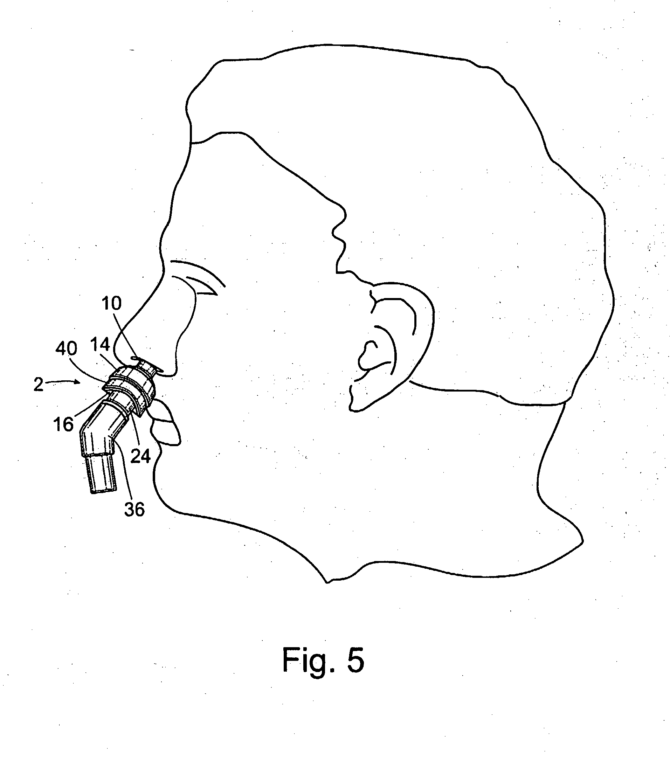

As shown in Figs. 2 and 3, embodiments of the present invention which include a sealing ring 40 can also include sealing tabs 42. Further, and as shown in Fig. 3, one embodiment of a nasal interface can also include one or more exhalation ports 22, which are described in more detail below. Lastly, Fig. 5 also shows a fully assembled nasal interface 2 properly positioned in the nares of a subject.

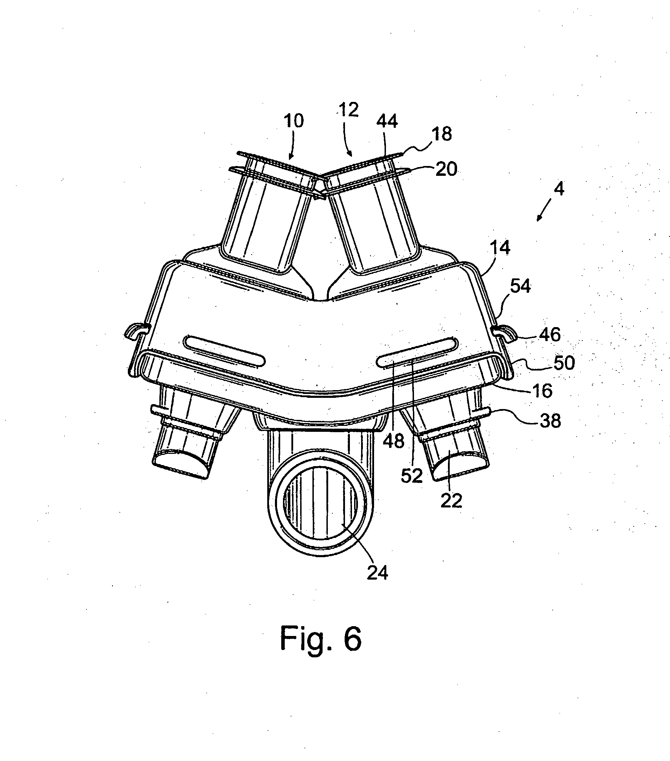

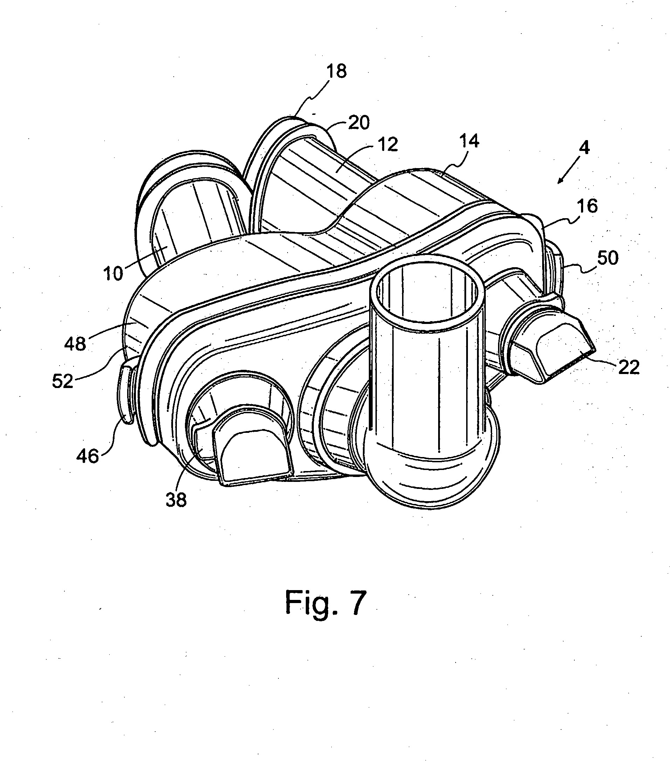

Figs. 6 and 7 show top and perspective views of another embodiment of the invention, including nasal interface body 4 that includes distal portion 14 and proximal portion 16, a first nasal prong 10 and a second nasal prong 12. In one embodiment, a nasal prong of the invention includes a first flap 18 and an optional second flap 20.

With respect to Figs. 6 and 7, to maintain a seal between proximal portion 14 and distal portion 16, proximal portion 14 of nasal interface 4 can also include one or more tab receiving openings 52 and hooked tab receiving openings 54. Distal portion 16 of nasal interface body 4 can also include one or more linking tabs 48 and hooked tabs 46 for connecting together the proximal portion 14 and distal portion 16, through tab receiving opening 52 and hooked tab receiving opening 54, respectively. Specifically, proximal portion 14 can be pulled over hooked tabs 46 by grip tabs 50 for assembly and disassembly of nasal interface body 4, and when assembled, the hooked tabs 46 and linking tabs 48 assist in the maintenance of a seal, as shown in Figs. 6 and 7, thereby sealing proximal portion 14 to distal portion 16 of nasal interface body 4 by a mechanical and/or friction fit.

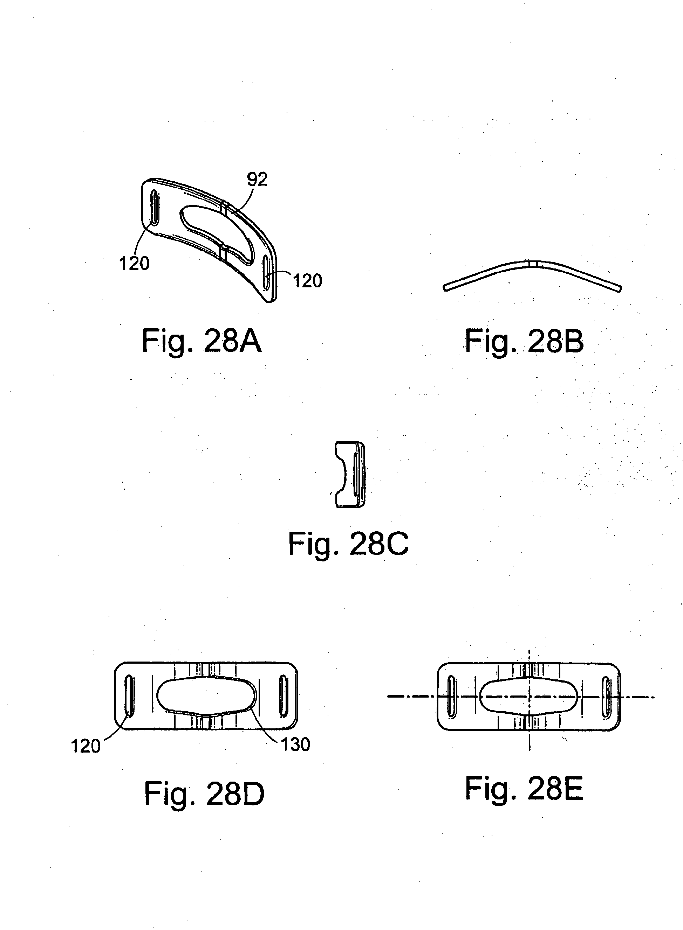

Nasal interface body 4 can also include inlet 24, which can be bent between 0 and 90 degrees up, as shown in Fig. 7 or 90 to 180 degrees down (not shown). A nasal interface body 4 can also include one or more exhalation ports 22 and locking tabs 38 to releasably engage a strap attachment plate 92, as shown in Figs. 28A through 28E.

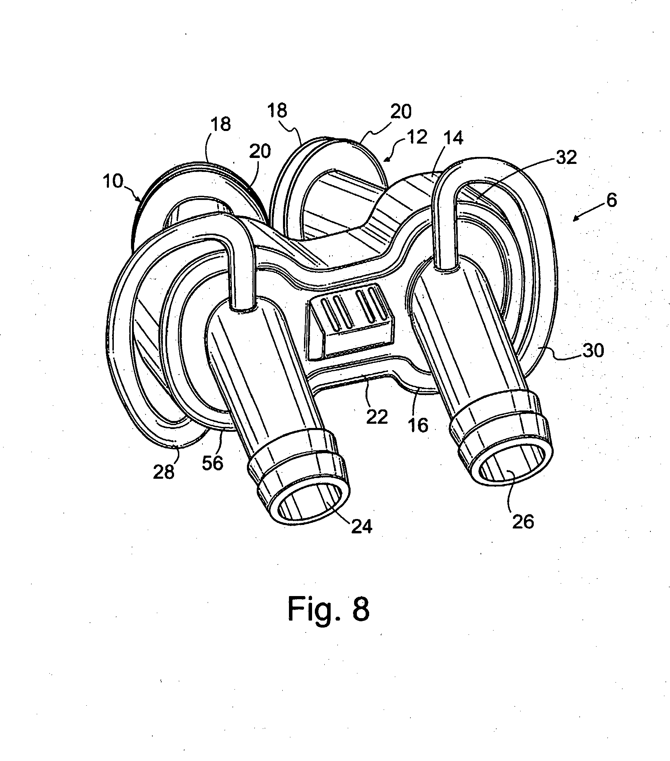

Fig. 8 provides a perspective view of another embodiment of the invention. To maintain a seal between proximal portion 14 and distal portion 16, proximal portion 14 includes a lip 56, which seals about the edge of distal portion 16, and thereby forming one embodiment of a nasal interface body of the present invention. The embodiment depicted in Fig. 8 further includes a first nasal prong 10 and a second nasal prong 12.

As shown in Fig. 8, nasal interface body 6 can also include first inlet 24 and a second inlet 26, as well as exhalation port 22. In one embodiment, exhalation port 22 is positioned between inlet 24 and second inlet 26.

To illustrate certain features and elements of the present invention, further details shown with respect to components and exploded views of certain embodiments are described hereafter.

### C. Exploded Views of Nasal Interfaces of the Present Invention

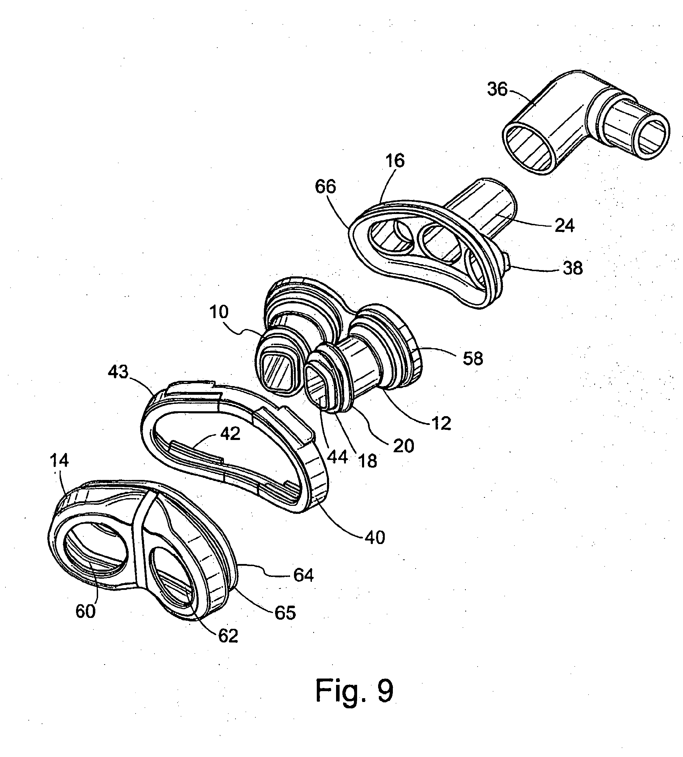

The exploded views of nasal interfaces of the present invention, which are set forth in Figs. 9 through 11, illustrate how the individual components of the invention can be combined together to form nasal interfaces of the invention. Accordingly, one of skill in the art can readily interchange components and features of different embodiments of the present invention in light of the teachings set forth herein to arrive at other embodiments. Such interchangeability is within the scope of this disclosure.

Fig. 9 shows an exploded view of the embodiment shown in Figs. 1 through 5, including a nasal interface 2 having a first nasal prong 10 and second nasal prong 12, proximal portion 14, distal portion 16, a sealing ring 40, sealing tabs 42, locking tabs 38, and a first flap 18 and optionally second flap 20 positioned on one or both of the nasal prongs.

As shown in Fig. 9, in one embodiment the nasal prongs 10 and 12 can be joined together by platform 58. With respect to proximal portion 14, openings 60 and 62, receive nasal prongs 10 and 12. Further, proximal portion 14 also includes a sealing extension 64, which can seat in sealing lip 66 of the distal portion 16.

In one embodiment, proximal portion 14 also includes a sealing ring groove 65, described further below.

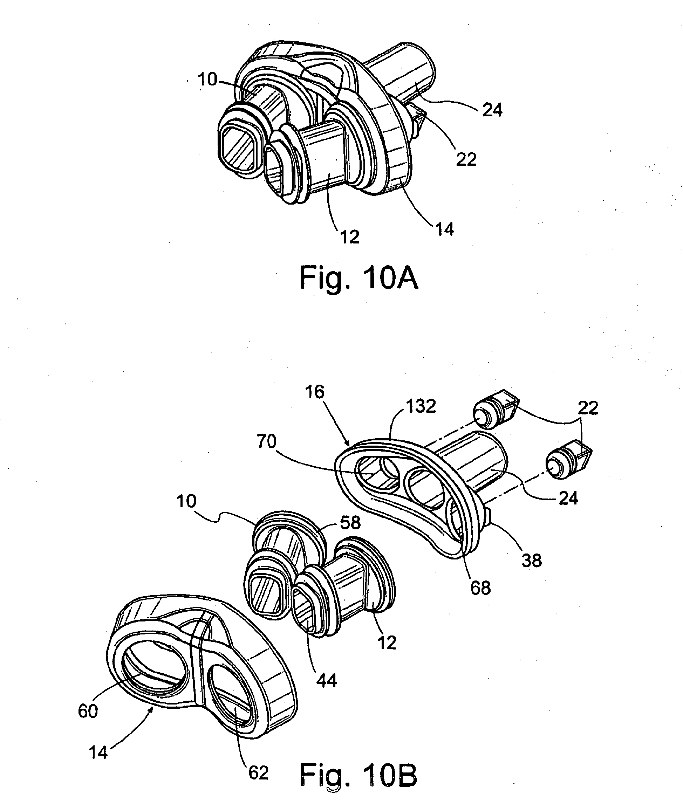

Figs. 10A and 10B illustrate another embodiment of the present invention. Fig. 10A illustrates a perspective view of a device of the present invention, having an inlet 24, exhalation ports 22, proximal portion 14, distal portion 16, and nasal prongs 10 and 12.

Fig. 10B shows an exploded view of Fig. 10A; illustrating a first nasal prong 10 and second nasal prong 12 joined by platform 58, proximal portion 14 and a distal portion 16. With respect to proximal portion 14, Fig. 10B also shows openings 60 and 62, for receiving the nasal prongs 10 and 12.

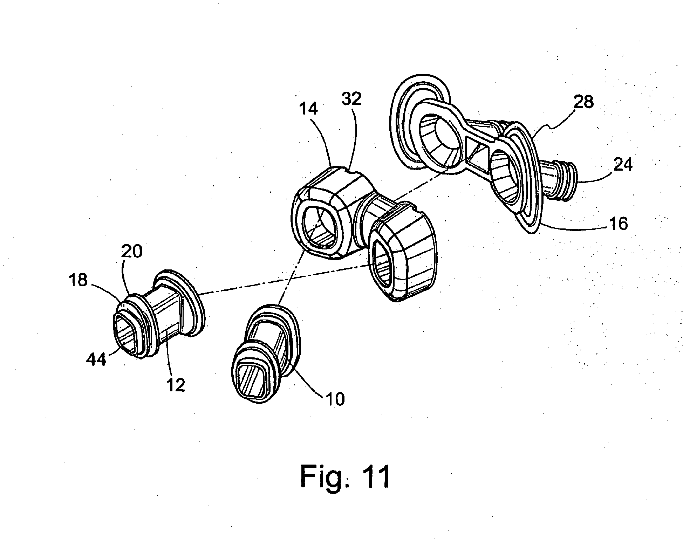

Fig. 11 shows an exploded view of the embodiment shown in Fig. 8, and includes a nasal interface having a first nasal prong 10, second nasal prong 12, proximal portion 14, distal portion 16 and inlet 24. Proximal portion 14 also includes an indent 32, as described in the following section.

### II. Components of the Nasal Interfaces of the Present Invention

Each component of the invention generally described above is described in more detail hereafter, including: A. proximal portion, B. distal portion, C. nasal prongs, D. exhalation ports, and E. sealing ring.

### A. The Proximal Portion

As noted above, "proximal portion" refers to a component of a nasal interface body that is closest to the user during use and typically contains openings for holding nasal prongs. In some embodiments, the proximal portion can be considered a "soft body" because of the materials which are used to make the proximal portion. In one embodiment, the proximal portion has a durometer of between about 10 to 80 on the Rockwell Hardness scale.

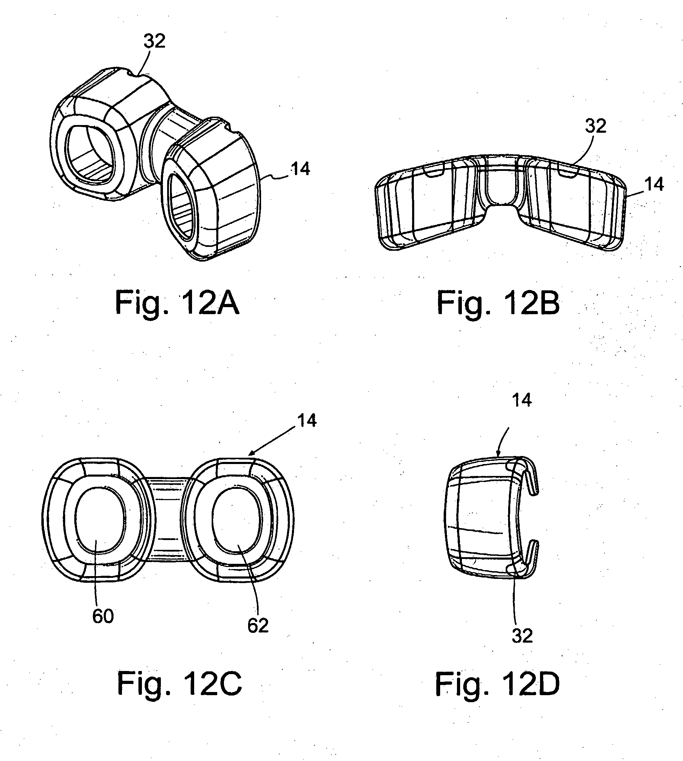

With respect to Figs. 12A through 13I, which depict different embodiments of proximal portions, common features are referred to with the same reference numbers and names.

Proximal portion 14 includes one or more openings 60 and 62. Openings 60 and 62 can be any regular or irregular shape, including round or circular, oval, rectangular or square to accommodate and/or hold one or more nasal prongs 10 and 12 securely in place. In one embodiment, the opening approximates the outside shape of the nasal prong that is to be placed in either opening 60 and 62. In one embodiment, opening 60 can be a different shape or different dimension (e.g., diameter if circular) than opening 62. Openings 60 and 62 can be positioned anywhere on proximal portion 14.

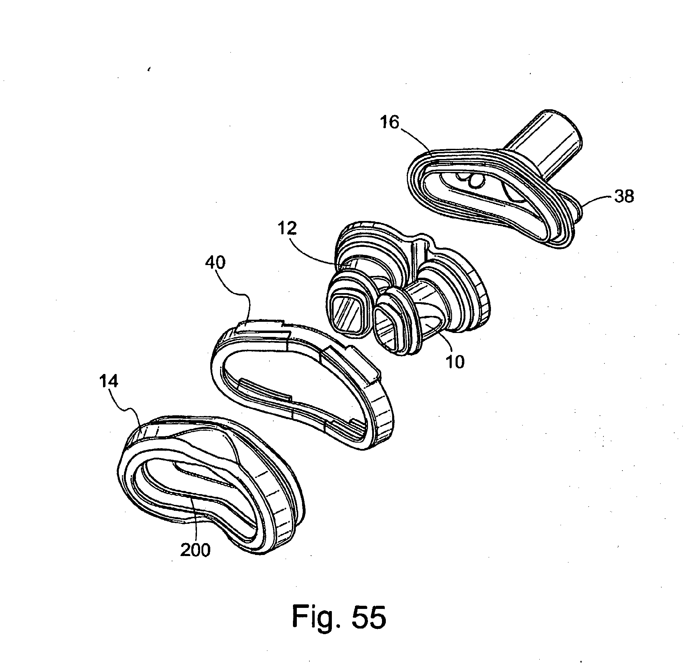

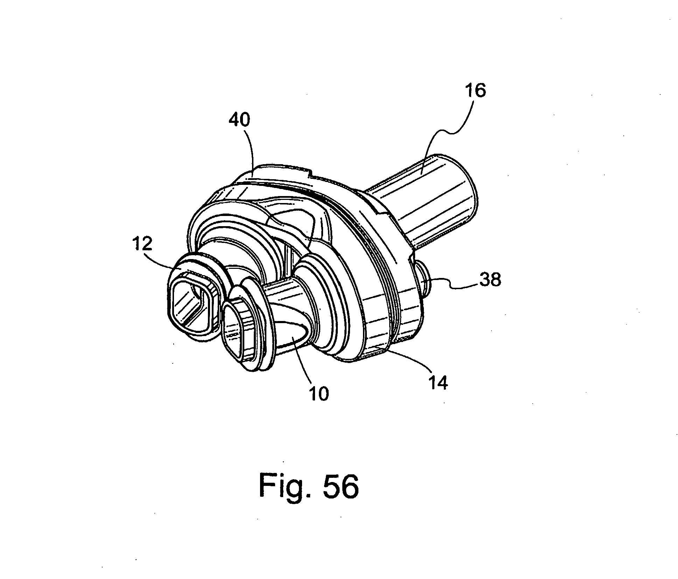

Further, in one embodiment, openings 60 and 62 can be positioned up to 5.0 inches apart. In one embodiment, openings 60 and 62 can be combined to make-up one larger opening, e.g., up to 5.0 inches wide to accommodate nasal prongs 10 and 12. In one embodiment, as shown by Figs. 55 and 56, openings 60 and 62 of proximal portion 14 can be replaced by a single opening 200. The single opening configuration shown in Figs. 55 and 56 can provide enhanced adjustments for the comfort and/or needs of a particular patient because different sized nasal prongs 10 and/or 12 can be substituted into a nasal interface of the invention without substituting proximal portion 14.

As shown in Figs. 12A, 12B and 12D, in one embodiment, proximal portion 14 includes one or more indents 32. Indents 32 are positioned on each side of the proximal portion 14 and can accommodate optional strap connections 28, which can be located on the distal portion 16 when assembled to distal portion 14, as depicted in Fig. 8 and discussed further below.

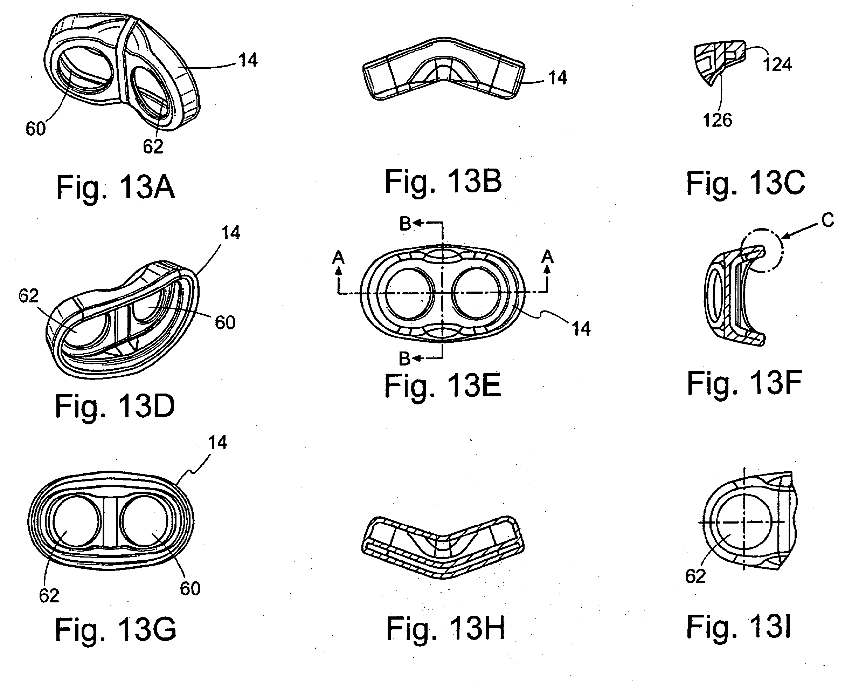

In certain embodiments, proximal portion 14 can be curved, as shown in Figs. 12B and 13B, so as to provide a shape that maximizes the comfort of the patient using an assembled nasal interface of the present invention.

In one embodiment, proximal portion 14 can be made out of a hard, rigid, flexible or soft material. In some embodiments, proximal portion 14 is made from hard plastic, such as polycarbonate, polyethylene, ceramic, acrylic, or any other material known in the art. In another embodiment, proximal portion 14 is made from a soft material, such as silicone, to enhance patient comfort when it contacts a patient's face. Preferably, the soft material used to form proximal portion 14 is of a higher durometer than the soft material of prongs 10 and 12, discussed below. As a result, proximal portion 14 is, in some embodiments, more rigid than nasal prongs 10 and 12. However, in one embodiment, proximal portion 14 is still flexible enough to be stretched over the edges of distal portion 16, forming a seal via proximal ridge 56, shown in Fig. 12D and Fig. 8 and/or via proximal ridges 124 and 126, shown in Figs. 13C and 13F. Proximal ridges 124 and 126 can seat around the edge of some embodiments of distal portion 16, thereby forming a seal and enhancing the fit between the components.

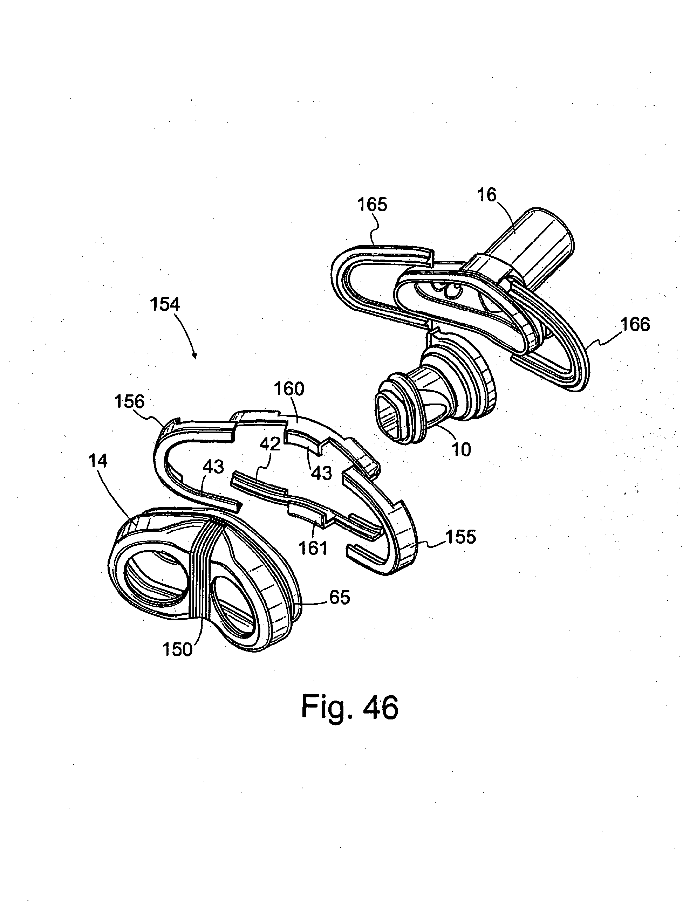

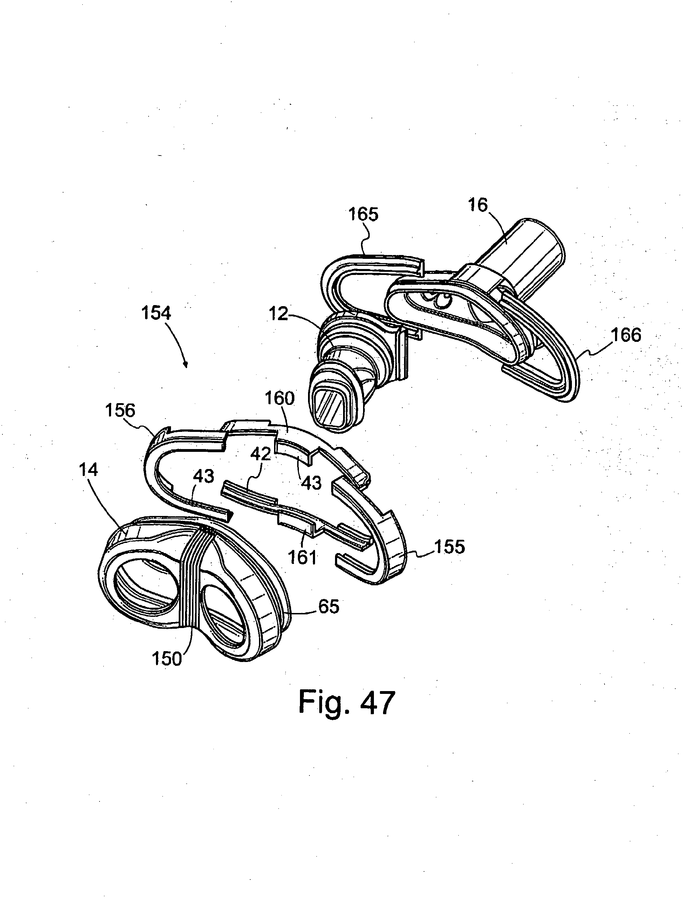

In one embodiment, proximal portion 14 includes a corrugated portion 150 or a portion of elastic or permanently deformable material to allow for adjustments of the distance between openings 60 and 62 to enhance the fit of the nasal prongs 10 and 12 in the nares of a patient, as shown in Figs. 46 through 48. Corrugated portion 150 can extend wholly or partially about proximal portion 14. In some embodiments, corrugated portion 150 can be positioned anywhere on proximal portion 14. In one embodiment, corrugated portion 150 can be positioned between openings 60 and 62, as shown in Figs. 46 through 48

In one embodiment, proximal portion 14 includes one or more tab receiving openings 52 and hooked tab receiving openings 54 so that proximal portion 14 can be stretched over distal portion 16 and releasably engage distal portion 16, as shown in Figs 6 and 7, thereby maintaining a seal via a mechanical and/or friction fit.

In another embodiment, proximal portion 14 includes a sealing groove 65, shown in Fig. 9, and sealing extension 64, also shown in Fig. 9. In one embodiment, groove 65 reversibly secures sealing ring 40 to proximal portion 14. By stretching sealing ring 40 over proximal portion 14, sealing ring lip 43 can seat in sealing groove 65, thereby forming a seal and removably securing sealing ring 40 to proximal portion 14, as shown in Fig. 1.

In one embodiment, exemplified by proximal portion 14 depicted in Fig. 9, sealing extension 64 is shaped to connect with sealing lip 66 of distal portion 16. Proximal portion 14 is then sealingly secured to distal portion 16 by a sealing ring 40 which fits into and/or connects to groove 65 of proximal portion 14, as described above and detailed further below.

In one embodiment, proximal portion 14 has a length of about 0.5 to 7.0 inches and a width of about 0.5 to 7.0 inches. Preferably, proximal portion 14 has a length of about 1.891 inches and a width of about 0.971 inches. However, proximal portion 14 may be of any size as long as it fits with distal portion 16 and securely holds nasal prongs 10 and 12 in openings 60 and 62.

### B. Distal Portion

As noted above, "distal portion" refers to a component of a nasal interface body that is furthest from the user during use and typically includes a gas inlet.

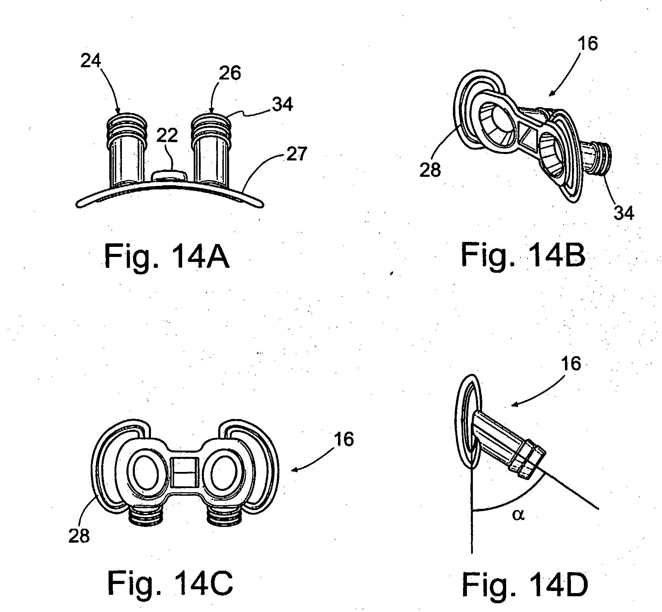

Specific embodiments of distal portion 16 are shown in Figs. 14A through 17H.

In one embodiment, distal portion 16 is made from any hard, rigid or flexible material. In some embodiments, the distal portion can have a higher durometer relative to the proximal portion. In one embodiment, distal portion 16 is made from hard plastic, such as polycarbonate, polyethylene, ceramic, acrylic, or any other material known in the art.

It should be noted that in order prevent carbon dioxide buildup, if an embodiment of the invention is off or not functioning, embodiments of the invention can have an inherent leak rate of gas from the device. In one embodiment, the use of polycarbonate for distal portion 16 will ensure a more consistent leak rate among all users.

In one embodiment, distal portion 16 is rigid to provide mechanical support for the rest of the nasal interface of the present invention.

Figs. 14A through 14D show an embodiment of distal portion 16 depicted generally in Fig. 8. As shown in Figs. 14A through 14D, distal portion 16 includes, in one embodiment, inlets 24 and 26, located on a back 27 of distal portion 16, opposite prongs 10 and 12 (also shown in Fig. 8).

Inlets 24 and 26 direct gas through a nasal interface to a subject's nares. In one embodiment, inlets 24 and 26 have one or more barb or ridge connectors 34 to receive gas supply tubing. Inlets 24 and 26 can have an inside diameter substantially equal to the inside diameter of the gas supply tubing in order to prevent any constriction or narrowing of the air passage which may cause increased velocity in air flow.

Preferably, when multiple inlets are employed, inlets 24 and 26 are located on distal portion 16 in parallel with one another and at an angle α, as shown in Fig. 14D. Angle α may range from about 0° to 180°.

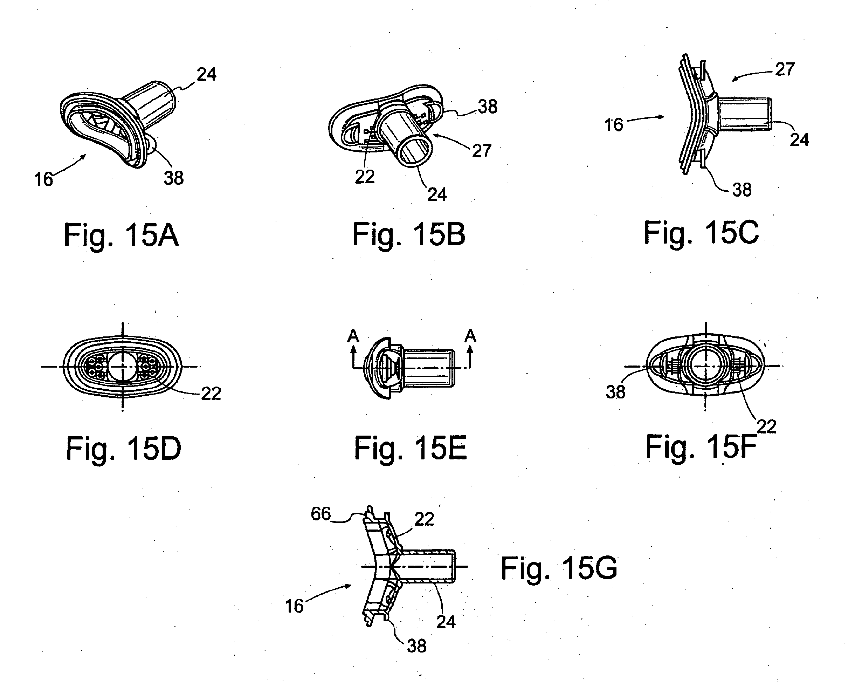

Although a gas inlet can be positioned on different components of the invention, as embodied by the invention depicted in Fig. 15A through 17H, distal portion 16 includes a single inlet 24, located on a back 27 of distal portion 16 opposite prongs 10 and 12 (as shown in Fig. 1). In such a single inlet configuration, the diameter of the single inlet can be larger than the diameter of inlets 24 or 26 to provide a flow capacity/resistance similar to the performance of both of the tubings in the two gas inlet configuration. A single gas inlet configuration can reduce resistance in the breathing circuit because the gas has less distance to travel from a ventilation machine. In one embodiment, the inner surface of inlets 24 and/or 26 and/or tubing 90 can be smooth to reduce resistant to gas flow.

As noted above with respect to the single inlet configuration, inlet 24 directs gas through an embodiment of a nasal interface of the invention to the nares. In one embodiment, inlet 24 is smooth to receive air supply tubing adapter 36. In another embodiment, inlet 24 has one or more barbs or ridges, as described above with respect to inlets 24 and 26.

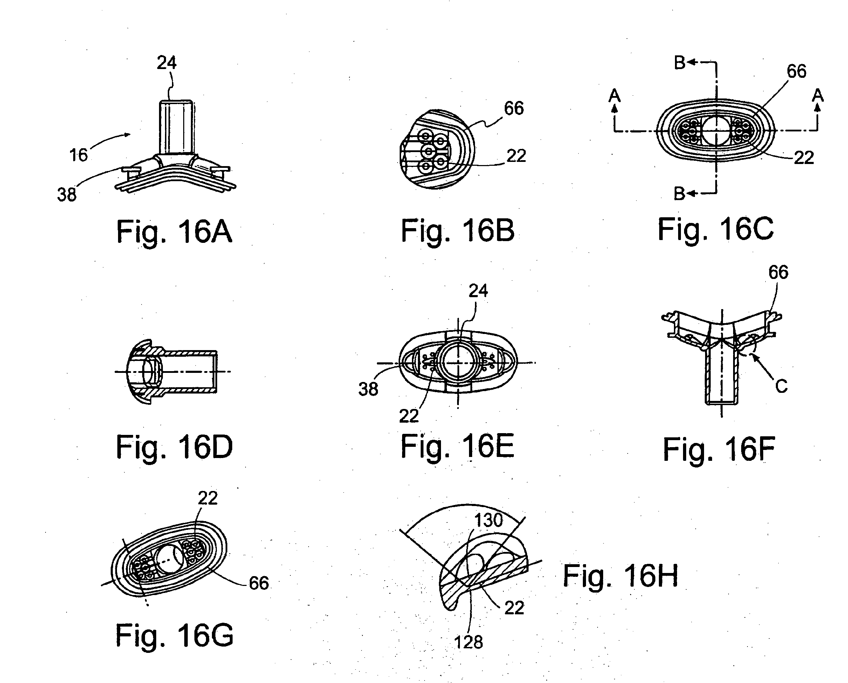

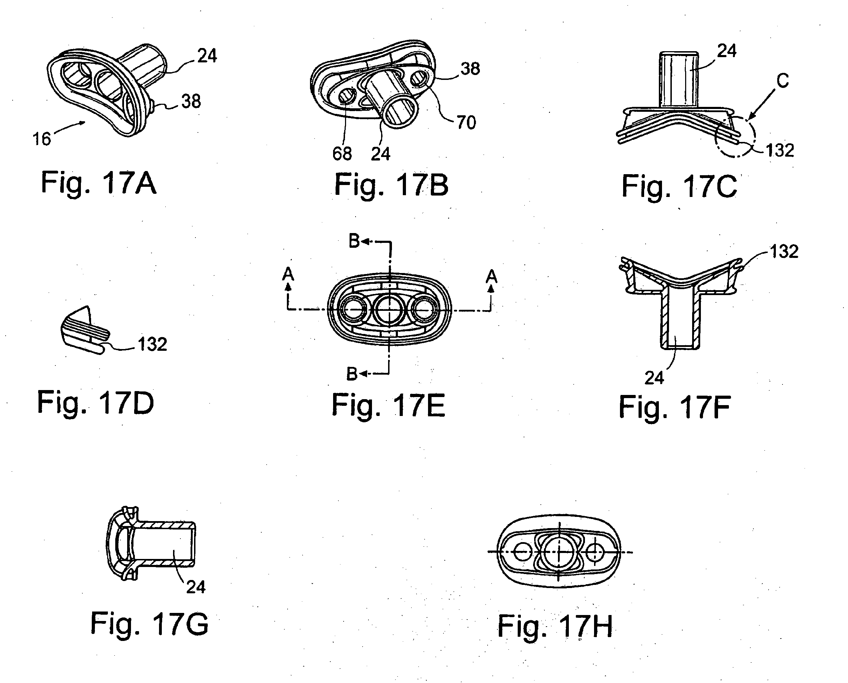

In some embodiments of the invention, as shown in Figs. 15G and 16F, distal portion 16 includes a sealing lip 66 for receiving sealing extension 64 of proximal portion 14, described above. In other embodiments, as shown in Fig. 17A through 17F, distal portion 16 (depicted in Fig. 10B) includes a distal portion receiving lip 132, which is shown in an enlarged view in Fig. 17D. Lip 132 is used in embodiments wherein proximal portion 14 is flexible and capable of stretching over distal portion 16, thereby forming a seal between distal portion 16 and proximal portion 14.

In another embodiment (e.g., as shown in Fig. 6) distal portion 16 includes linking tabs 48 and hooked tabs 46 for connecting together proximal portion 14 and distal portion 16. As noted above, proximal portion 14 can be pulled over hooked tabs 46 by grip tabs 50 for assembly and disassembly of a nasal interface, and when assembled, hooked tabs 46 and linking tabs 48 assist in the maintenance of a seal, as shown in Figs. 6 and 7.

As shown in the figures and in particular Figs. 3, 14A, 15D, 15F, 16C and 16F, distal portion 16 can also include one or more exhalation ports 22, described further below.

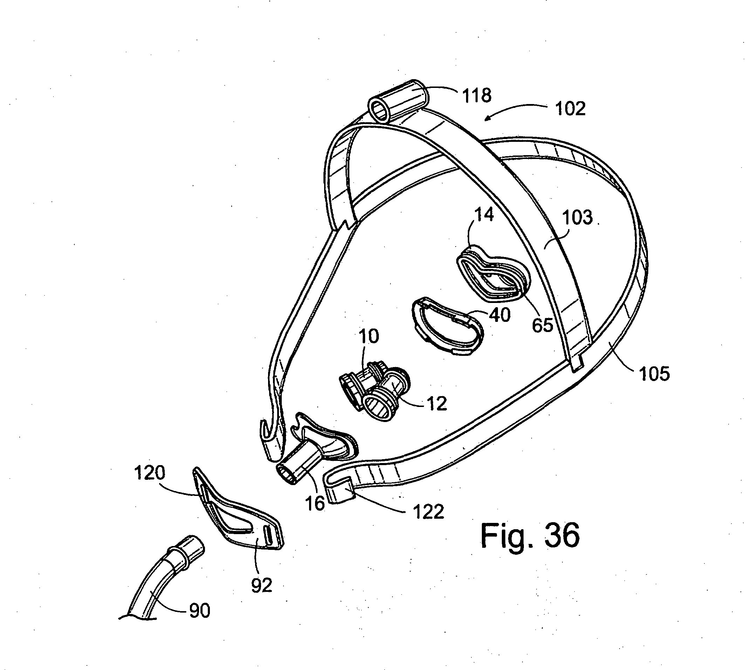

In one embodiment, distal portion 16 can also include one or more locking tabs 38, (e.g., as shown in Figs. 1 through 3, 6, 15A, 15B, 15G and others) for securing a strap attachment plate 92 (described below with respect to Figs. 36 and 37) to distal portion 16, in the manner shown in Fig 37.

In one embodiment, distal portion 16 can be about 0.5 to 7.0 inches wide, typically about 2.2 inches wide. Distal portion 16 can be about 0.8 to about 1.5 inches tall, typically about 1.2 inches tall. Distal portion 16 can also be about 0.5 to 7.0 inches long typically about 1.5 inches long.

### C. Nasal Prongs

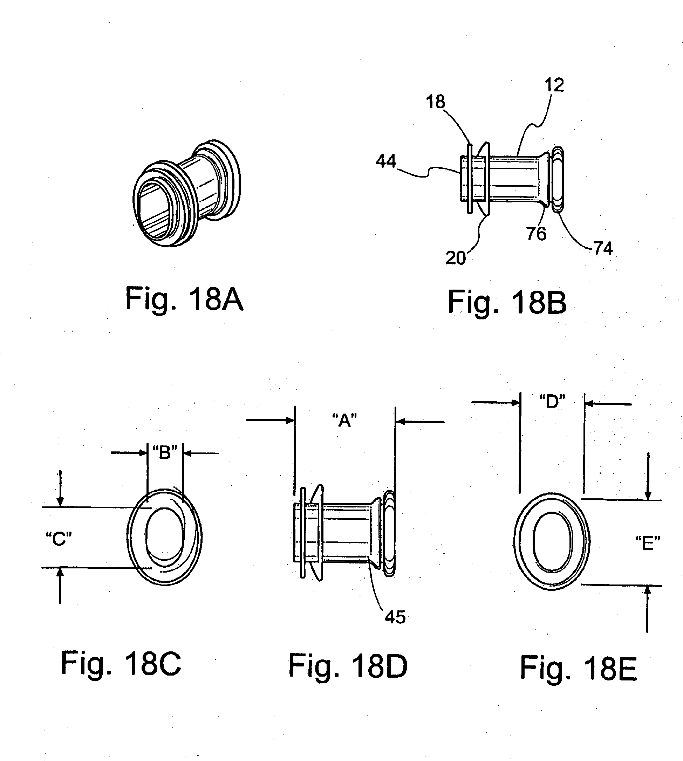

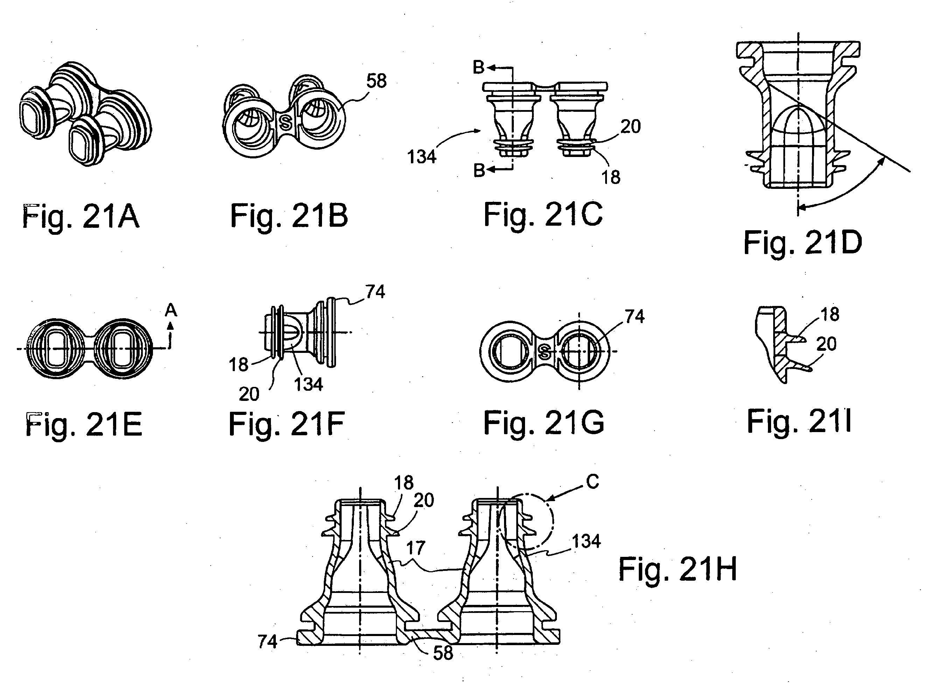

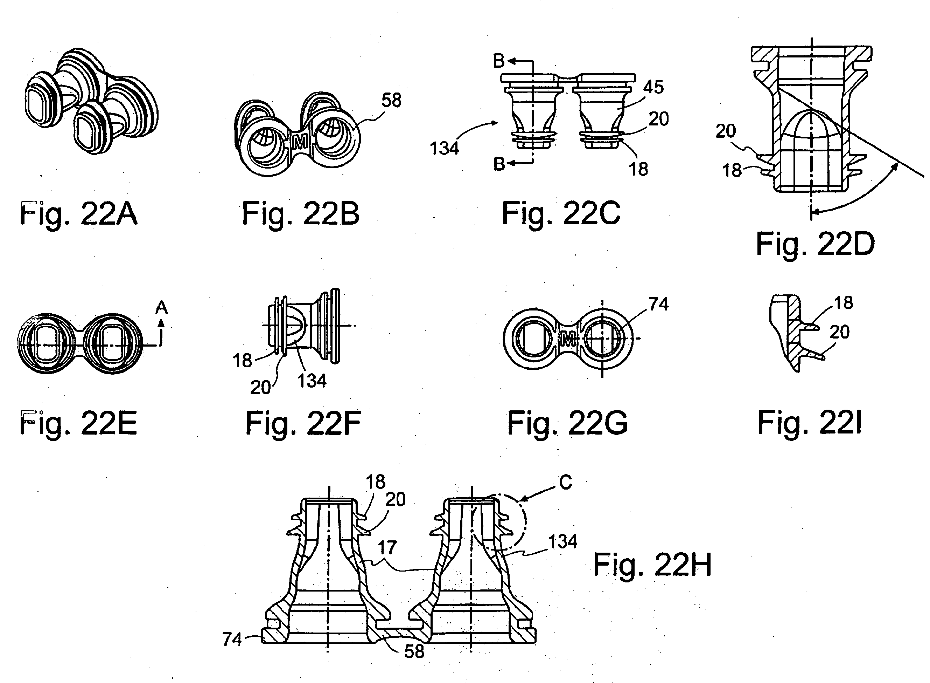

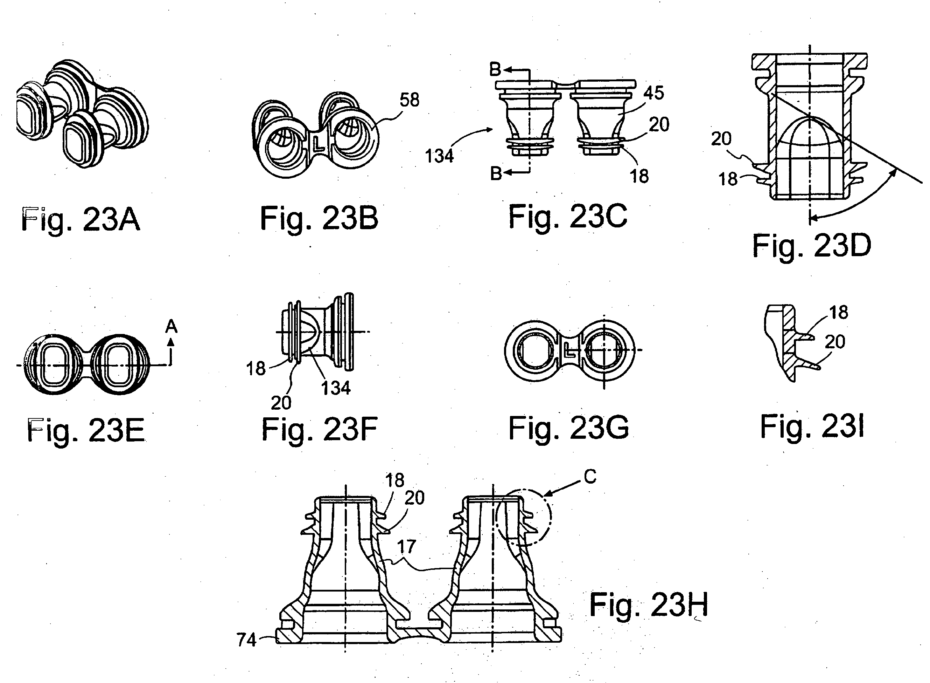

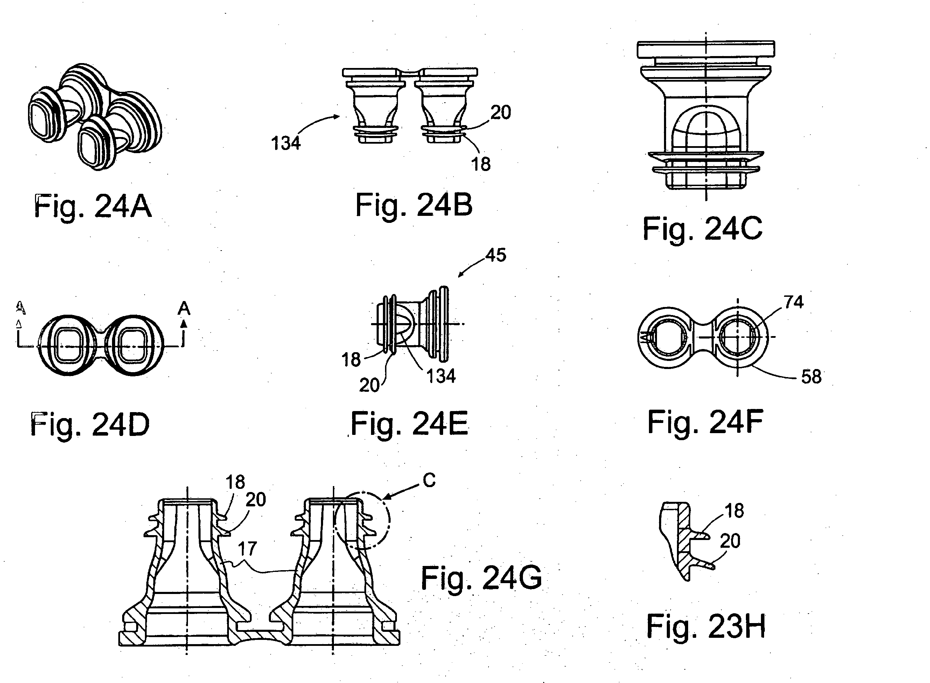

Nasal prongs 10 and 12, shown in Figs. 18A through 24H can be any regular or irregular cross sectional shape and may change in shape along the length of the prong. Preferably, nasal prongs 10 and 12 are oval or rectangular in cross sectional shape. Nasal prongs 10 and 12 are sized to fit various patient nares (e.g., sizes XS to XL) and can removably attached to proximal portion 14, as described further below. For example, as discussed below, nasal prongs 10 and 12 can be supplied to a user in sizes from extra small to larger or more, when the inner and/or outer diameter of nasal prongs 10 and 12 are sized to fit different size nares.

Nasal prongs 10 and 12 are hollow, each having a bore 11. Bore 11 is a longitudinal opening through the length of each nasal prong, is defined by the material (e.g., silicone) of the nasal prong, and forms a continuous flow path or conduit for the passage of inhaled and exhaled gases between the patient's nares and the nasal interface of the present invention such that the exhaled gas can be expelled front the nasal interface through an exhalation port, which are described in greater detail in the next section. Bore 11 can be the same or different shape than the shape of nasal prongs 10 and 12. For example, nasal prongs 10 and 12 can have a round shape, whereas bore 11 can be oval in shape.

Nasal prongs 10 and 12 typically have the same, but may also be of different dimensions, depending on the patient's nares. In the embodiments that include nasal prongs having oval or rectangular portions, the various dimensions may include length A, inner short diameter B, inner long diameter C, outer short diameter D and outer long diameter E, as shown in Figs. 18C through 18D. Table 1 below shows several examples of the various dimensions for nasal prongs 10 and 12.

**Table 1**

| **Dimensions, inches** | | | | | |
|---|---|---|---|---|---|
| **Example** | **A** | **B** | **C** | **D** | **E** |
| 1 | 0.900 | 0.468 | 0.652 | 0.584 | 0.704 |
| 2 | 0.900 | 0.276 | 0.472 | 0.584 | 0.704 |
| 3 | 0.900 | 0.290 | 0.434 | 0.584 | 0.704 |
| 4 | 0.900 | 0.264 | 0.395 | 0.584 | 0.704 |
| 5 | 0.900 | 0.237 | 0.355 | 0.584 | 0.704 |

In those embodiments that include nasal prongs having round portions, the diameter can range from about 0.3 to about 0.6 inches.

The above dimensions are exemplary, and the actual dimensions of nasal prongs 10 and 12 may vary. In one embodiment, dimensions A, D and E remain constant with each nasal interface of the present invention so that prongs 10 and 12 of different sizes may be interchanged with other prongs of the invention. In some embodiments, nasal prongs 10 and 12 can be made from soft, and/or flexible, and/or resilient, and/or biocompatible materials. Preferably, nasal prongs 10 and 12 are made from a soft, pliable material, such as silicone, urethane, polyvinyl chloride, santoprene, medical rubbers and similar thermoplastic materials. This allows a user to change prongs 10 and 12, and to use a suitable size, depending on the dimension of the user's nares.

Fig. 18A provides a perspective view of one embodiment of the nasal prong of the invention. Fig. 18D, which is a 90-degree rotation of Fig. 18B, illustrates the optional difference in dimensions between measurements "D" and "E" described above.

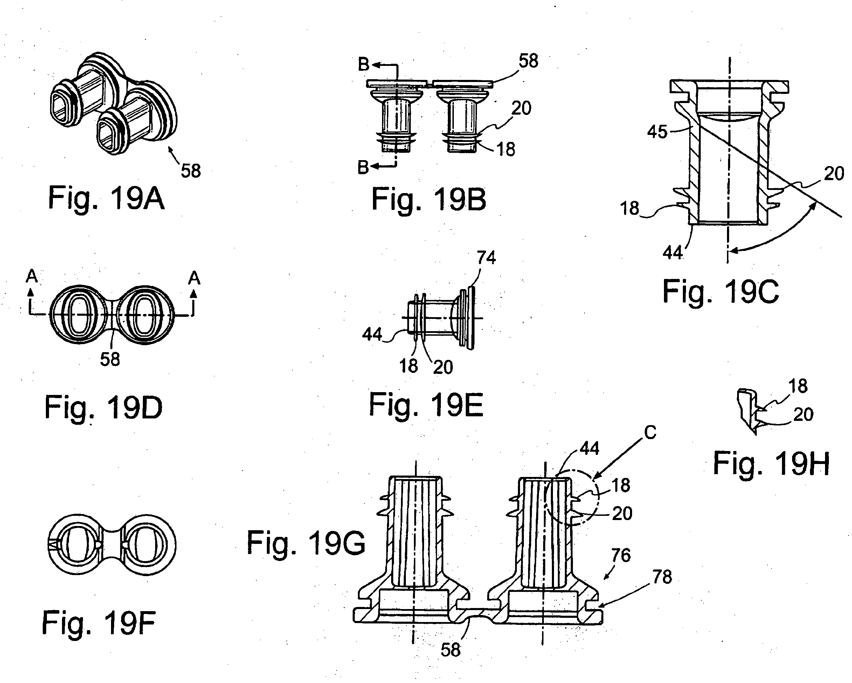

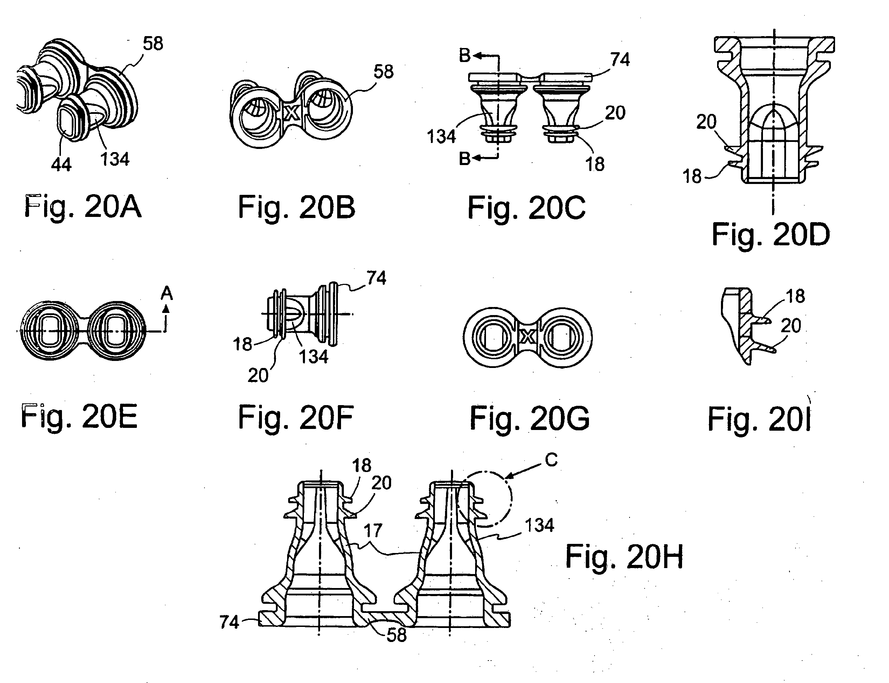

In one embodiment, nasal prongs 10 and 12 have a constant cross sectional shape over a longitudinal length (i.e., along the long axis or axially) of the prongs, as shown, for example, in Figs. 18B, 18D, 19B and 19G. In other embodiments, nasal prongs 10 and 12 have a first cross sectional shape and/or cross sectional area at second end 74 (also referred to herein as end 74) and a different cross sectional shape and/or cross sectional area at first end of the prong, tip 44 (which is referred to herein as "the prong tip 44"). In one embodiment, nasal prongs 10 and 12 have a round or circular cross section at second end 74 and oval or rectangular cross section at a first end, e.g., prong tip 44, with a transition from one cross section to another (e.g., round to oval or rectangular) at nasal prong taper 134 (see, e.g., Figs. 20C, 20F, 21C, 21F, 22C, 22F, 23C, 23F, 24C, and 24E). In other embodiments, the nasal prongs 10 and 12 of the present invention can undergo more than one transition in shape or diameter.

In embodiments that include a transition in cross sectional shape and/or cross sectional area from second end 74 to the first end (i.e., prong tip 44), the various dimensions may include length A, first inner short diameter F, first inner long diameter G, second inner short diameter H and second inner long diameter I, wherein F and G are the dimensions of second end 74 and H and I are the dimensions of prong tip 44. The following examples include embodiments that have a round or circular cross sectional second end 74. In some embodiments, the F and G dimensions are the same for each example.

**Table 2**

| **Dimensions, inches** | | | | | |
|---|---|---|---|---|---|
| **Example** | **A** | **F** | **G** | **H** | **I** |
| 6 | 0.900 | 0.377 | 0.377 | 0.245 | 0.377 |
| 7 | 0.900 | 0.433 | 0.433 | 0.275 | 0.433 |
| 8 | 0.900 | 0.472 | 0.472 | 0.310 | 0.472 |
| 9 | 0.900 | 0.515 | 0.515 | 0.350 | 0.515 |

In some embodiments, a nasal prong of the present invention includes one or more nare sealing portions about (i.e., around) the first end. In one embodiment, the sealing portions include flaps 18, 20 shown, for example, in Figs. 1, 2, 4, 6, 7, 8, 9, 11, 18A-E, 19AH, 20A-I, 21A-I and 22A-I, 23A-I, 24A-H, and 30. In certain embodiments, the at least one flap (e.g. 18 or 20) is located proximate to (i.e., at or near) prong tip 44. The location of a flap near a prong tip 44 can create a seal between the nasal prong and nares of a patient (not shown). In some embodiments, the anatomy of the nose may deform in response to pressure from the inserts and flap(s) 18, 20. Preferred insert durometer is 20 to 40 hardness, shore A, with a possible durometer range of 10 to 70 hardness, shore A. Preferred flap thickness is about 0.020 inches, with a possible range of 0.005 to 0.50 inches. Flap 18 can extend out to a distance of 0.125 inches away from prong tip 44, with a distance range from 0.005 to 1.0 inches.

In one embodiment, shown in Fig. 6, the nare sealing portion (e.g., flap 18, 20) can be positioned at or about prong tip 44 of a nasal prong. In other embodiments, as depicted, for example, in Figs. 1, 2, 9, 11, 18A-E, 19A-H, 20A-I, 21A-I and 22A-I, 23A-I, 24A-H, and 30, nare sealing portion (e.g., flap 18, 20) can be positioned proximate to, near or below, but not at or about, prong tip 44 of a nasal prong. As shown generally in Figs. 1, 19H, 20I, 21I, 22I, and 23I, a nare sealing portion (e.g., flap 18) can be positioned between about 0.05 to about 0.30 inches, typically about 0.1 inches, from first end (e.g., prong tip 44), depending on the size of the nasal prong and needs of the user. In one preferred aspect of the invention, the nare sealing portions (flaps 18, 20) deform (e.g., fold over) to form to the curvature of the inner walls of the nares, thereby creating a seal.

There are several advantages to having deformable flaps 18, 20 on the nasal prongs 10, 12. First, an airtight seal is created within the nares just by action of the deformable flaps 18, 20. No additional radial expansion or pressure is needed to form a good seal within the nares of a patient. This is important because a seal within the nares is maintained even at low flow rates (or no flow rate at all) of air within the device 2. Second, patient comfort is greatly enhanced because of the relatively small amount of material forming the flaps 18, 20. There is no need for bulky or otherwise obtrusive sealing structures on the prongs 10, 12. Instead, the flaps 18, 20 of the device 2 form a seal within the patient's nares by gently deforming to the contour of the inner wall of the nares.

Although a single nare sealing portion (e.g., flap 18) can be used, in some embodiments one or more additional nare sealing portions can be positioned proximate to, near or below the first sealing portion. As shown in Figs. 1, 2, 4, 6, 7, 8, 9, 11, 18A-E, 19AH, 20A-I, 21A-I and 22A-I, 23A-I, 24A-H, and 30, nasal prongs 10 and 12 of the invention include a second nare sealing portion (e.g., flap 20). The second sealing portion (e.g., flap 20) can be positioned about 0.6 to 0.01 inches from the first sealing portion (e.g., flap 18). Separate nasal prongs also allow the size and/or diameter of one nasal prong to be different from the other nasal prong (e.g. the size of prong 10 is "small" and the size of prong 12 is "extra small"). This is particularly important for patients suffering from a deviated septum. In this patient population, it is common for patients to have different sized nares (i.e., left and right nares have different anatomical shapes). In the present device 2, different sized nasal prongs 10, 12 may be interchanged into the proximal portion 14 of the nasal interface body.

Nare sealing portions, (e.g., flaps 18 and/or 20) can also have an attachment angle from 0 degrees to more than 30 degrees, typically about 20 degrees, as depicted in Figs. 19H, 20I, 21I, 22I, and 23I. In one embodiment, the attachment angle causes flaps 18 and/or 20 to have a downward slope. In another embodiment, the attachment can have no slope (e.g., level) or have an upward slope. The attachment angle can increase the level of comfort of the nasal prong and/or the amount of seal when the prong of the present invention is placed or positioned in a nare. In one embodiment, the width of the first flap 18 is smaller than the width of the second flap 20. In another embodiment, the width of the first flap 18 is the same or greater than the width of the second flap 20.

Nare sealing portions (e.g., flaps 18 and/or 20) can also have a variation in thickness as the flaps extend radially outward from nasal prong bore 11. In one embodiment, the nare sealing portions (e.g., flaps 18 and/or 20) become thinner as they extend radially outward. In another embodiment, the nare sealing portions become thicker as they extend radially outward.

Nare sealing portions (e.g., flaps 18 and/or 20) can also vary in size and cross sectional shape and do not necessarily correspond to the size and/or shape of bore 11 or the shape of the prongs 10 and 12 to which one or more nare sealing portions are attached. For example, in one embodiment, a nare sealing portion can be round and the attached prong 10 can be oval, and vice versa. In another embodiment, a nare sealing portion can be oval and prong 10 can be rectangular in shape, and vice versa. Preferably, the flaps 18, 20 have an oval shape to better conform to the nares of the patient.

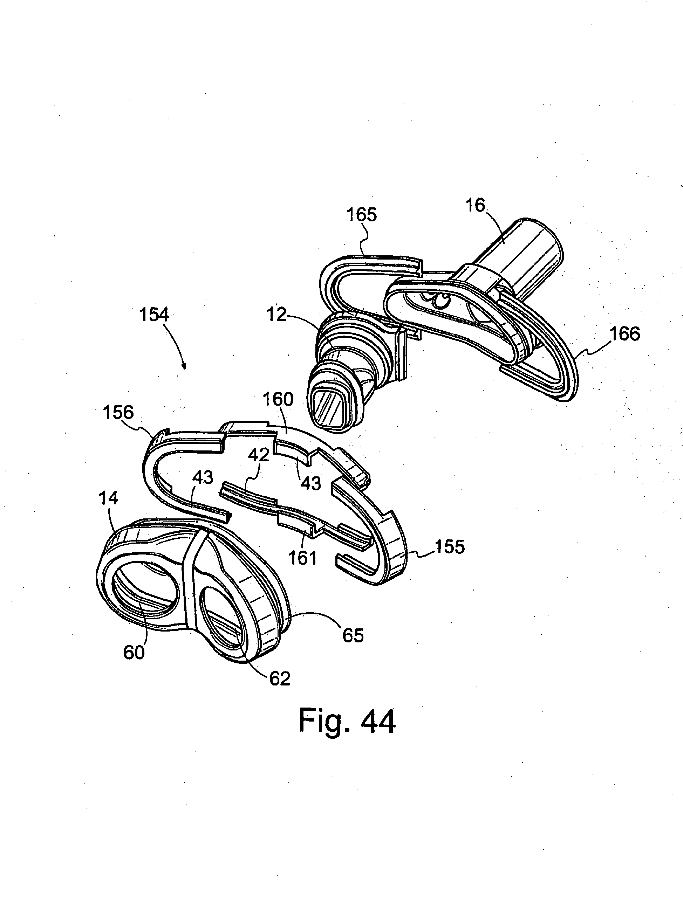

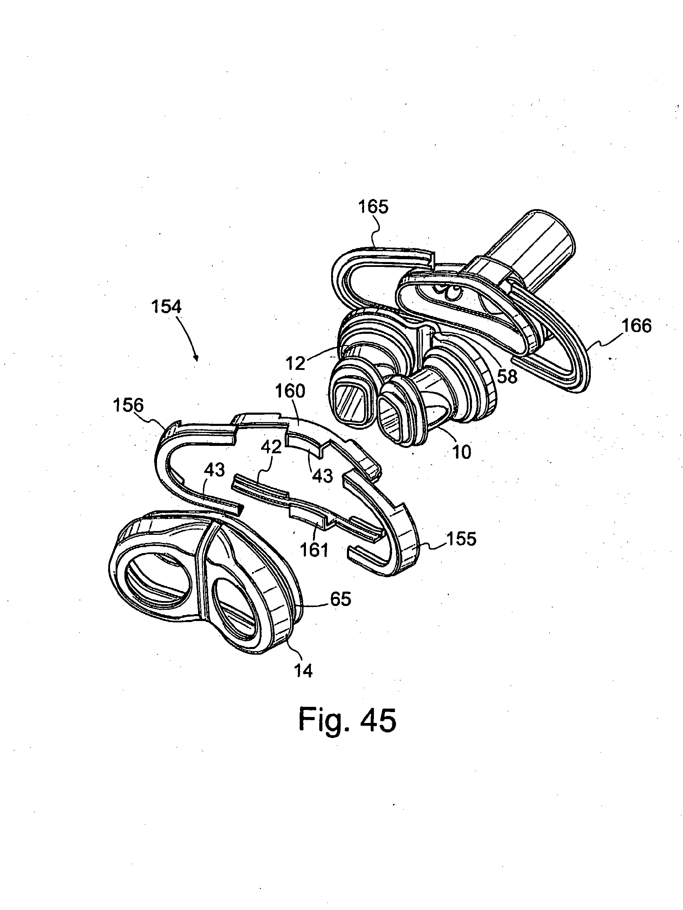

As seen in Figs. 1, 20H, 21H, 22H, 23H, 24G, 43-50, 51A, 51B, 55-60, in one preferred aspect of the invention, the nasal prongs 10, 12 include a shoulder portion 17 (shown in Figs. 20H, 21H, 22H, 23H, 24G) which is advantageously angled or tapered and acts as an abutment or stop for the nares of the patient. In particular, the shoulder portion 17 of the nasal prongs 10, 12 prevents the nasal prongs 10, 12 from being inserted too deeply within the nares. The movement of the nasal prongs 10, 12 within the nares is limited when the nasal wall abuts or comes into contact with the shoulders 17.

In some embodiments, nasal prongs 10 and 12 can be separate nasal prongs, as shown by the single nasal prong depicted in Fig. 18A, and Figs. 43, 44, 46, 47. In one embodiment, nasal prong 10 is a different size and/or shape and/or includes one or more dimensions that are different than nasal prong 12. In such embodiments, using different sized or shaped nasal prongs provides the advantage of enhanced sealing and comfort of the prongs in the nares of a patient as well as permitting a nasal prong to be interchanged with another nasal prong which may provide further enhanced sealing and/or comfort.

Additionally, by employing separate nasal prongs, either nasal prong 10 and 12 can be rotated and/or adapted to be rotatable in the proximal portion 14 opening or aperture (e.g., openings 60 or 62) in which the prong is positioned and/or releasably engaged, independenf of the other nasal prong to thereby enhance patient comfort and/or sealing against the nares of the patient. In preferred rotatable arrangements, it is preferred that the nasal prongs maintain a seal or remain substantially sealingly engaged with proximal portion 14.

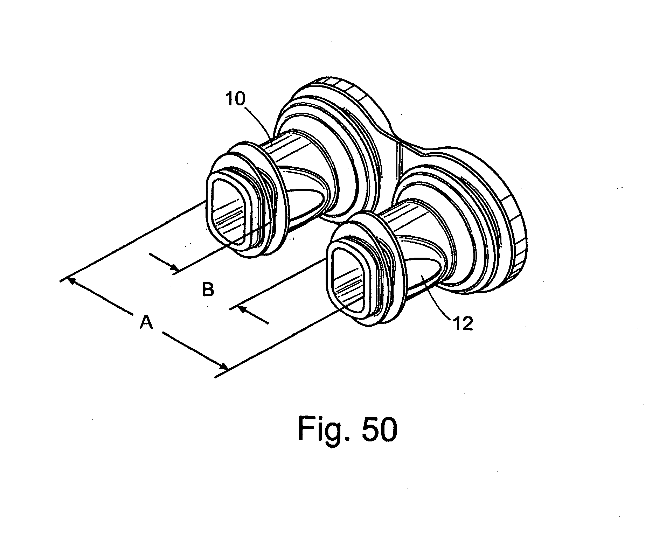

Although nasal prongs for use in the present invention can be separate components, as shown by the single nasal prong depicted in Fig. 18A, in other embodiments, prongs 10 and 12 can be optionally joined together by platform 58, as shown in Figs. 9, 19G, 20H, 21H, 22H, 23H, and 24H and in perspective views 19A, 20B, 21B, 22B, 23B, and 24A. In such embodiments, prongs 10 can be the same or different size or shape relative to prong 12. The distance between the nasal prongs is typically 0.10 to 1.0 inches, from the outside edges of ends 74 to match the spacing of a patients nares, as shown by measurement "A" in Fig. 50B.

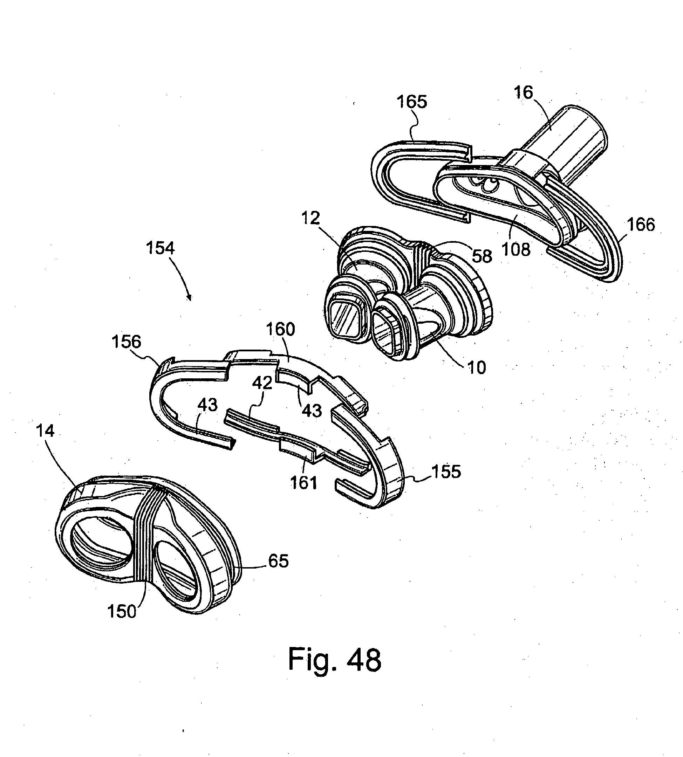

Platform 58 can optionally include a letter designation for an indication of a size of the nasal prongs, as shown in Fig. 19F, 20G, 21G, 22G, 23G, and 24F. Additionally, in some embodiments, platform 58 can be corrugated, elastic or permanently deformable to permit adjustments of the distance between the nasal prongs 10 and 12, as shown in Fig. 48. Finally, platform 58 may be formed from a cuttable material. More particularly, the platform 58 may be cut or otherwise separated into two halves, with each half containing a nasal prong 10, 12. In this aspect, the platform 58 is formed from a soft, pliable material that can be cut by a tool such as, for example, scissors.

In one embodiment, the distance between joined nasal prongs 10 and 12 can correspond to the distance between the nares of a patient in need of the present invention. In another embodiment, the distance between nasal prongs 10 and 12 can be varied. In one embodiment of the present invention, platform 58 can be a corrugated material or elastic, inelastic, or permanently deformable material to permit the adjustment of the distance between nasal prongs to enhance the fit of the nasal prongs 10 and 12 in a proximal portion 14 and ultimately in the nares of a patient, as described above and shown with respect to Figs. 46 through 49.

In one embodiment, with respect to nasal prongs 10 and 12, an end 74 opposite prong tip 44 connects with proximal portion 14 (shown in Fig. 2) by inserting ridge 76 (shown in Figs. 18B and 19G) through openings 60 or 62 in proximal portion 14, such that proximal portion 14 is secured, preferably sealingly secured, in lip 78, between ridge 76 and end 74 and the remaining part of nasal prongs 10 and 12 project from openings 60 and 62, as shown in Fig. 1.

In another embodiment, nasal prongs 10 and 12 attach to proximal portion 14 by sliding end 74 through openings 60 and 62 such that the ends are held within openings 60 and 62 of the nasal interface, while the remaining part of nasal prongs 10 and 12 project from openings 60 and 62, in the manner shown in Fig. 11.

In one embodiment, nasal prongs 10 and 12 and proximal portion 14 can be combined to form part of a nasal interface of the present invention. In another embodiment, nasal prongs 10 and 12 and proximal portion 14 integral with each other and form part of a nasal interface of the present invention. In embodiments which include a single body and interchangeable nasal inserts (i.e., nasal prongs 10 and 12 are not integral with proximal portion 14), the manufacturing costs can be significantly reduced because of easier manufacturing techniques.

In one embodiment, nasal prong 10 and/or 12 can be compressed and inserted into a patient's nares. In one embodiment, nasal prong 10 and/or 12 can be retained in the patient's nares solely by the sealing portion (e.g., flaps 18 and/or 20),

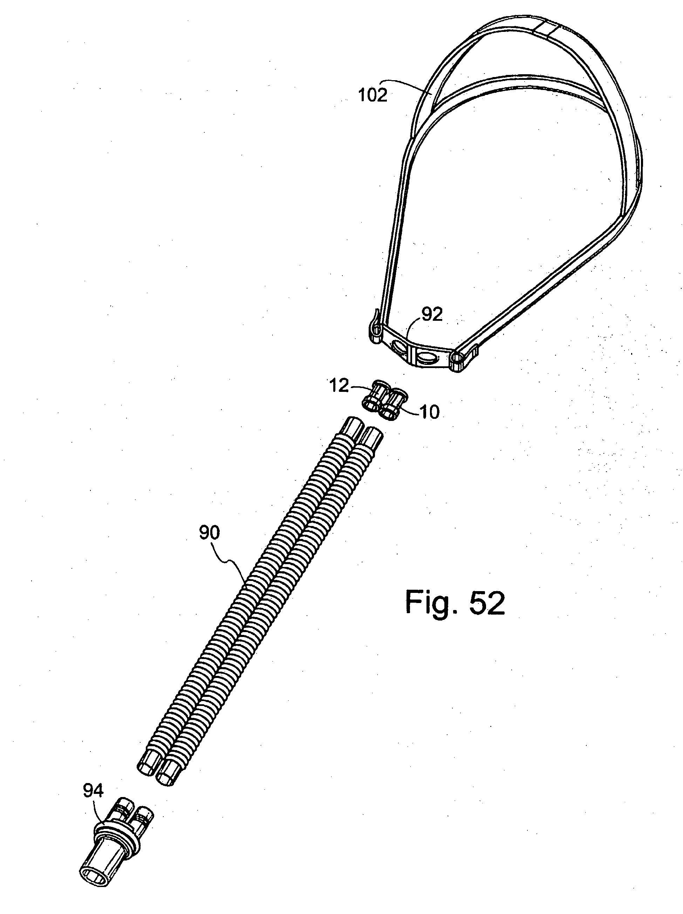

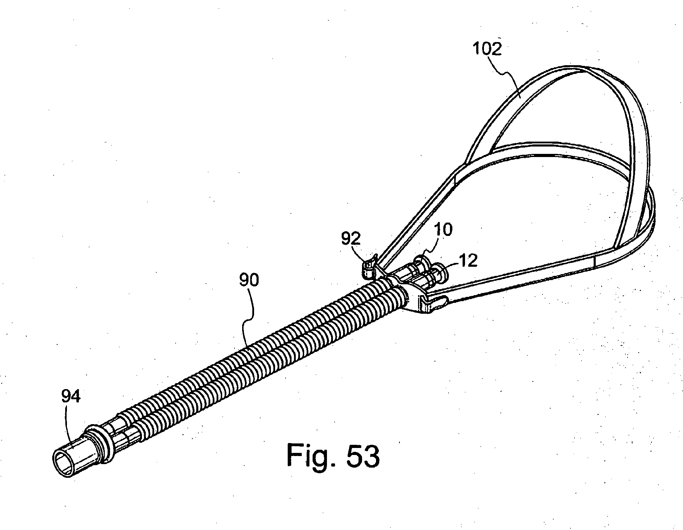

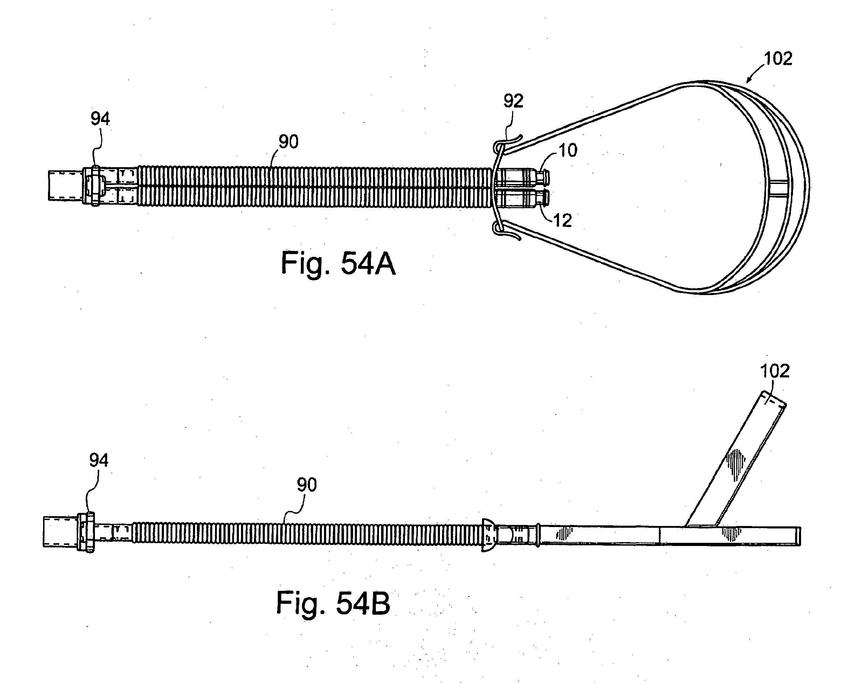

In some embodiments of the invention, the nasal prongs 10 and 12 can be directly connected to a ventilator apparatus via additional tubing or the like. The ventilator apparatus can be used to provide air, enriched air (about 22% to 100% Oxygen), or alternative gas mixtures to a patient in need thereof directly through nasal prongs 10 and 12. As shown in Figs. 52 through 54, nasal prong 10 and/or 12 can be directly connected to a ventilator via flexible tubing 90. In one embodiment, nasal prongs 10 and 12 can releasably engage plate 92 and flexible tubing 90, which in turn can be secured to a patient's head by virtue of strap system 102. In another embodiment, as shown in Figs. 52 and 53, plate 92 can secure flexible tubing 90 by inserting flexible tubing 90 into plate 92. In one embodiment, nasal prongs 10 and/or 12 can be releasably engaged with flexible tubing 90 by inserting flexible tubing 90 into second end 110 and/or 112 of nasal prongs 10 and/or 12.

### D. Exhalation Ports

In one embodiment, exhalation ports may be located on any portion of the body of the nasal interface of the present invention, including distal portion 16 and/or proximal portion 14. The exhalation ports for use in the present invention may be of any size or regular or irregular shape, but are preferably of a size and shape to allow exhaled gas to exit the device such that the carbon dioxide is sufficiently purged from the nasal interface.

In one embodiment, as shown in Figs. 3 and 8, exhalation ports 22 can be a series of parallel apertures located on distal portion 16.

In another embodiment, as shown in Figs. 15A through 16H, the invention can have one or more exhalation ports 22 in the form of holes or bores in distal portion 16. In one embodiment, exhalation port 22 can be any regular or irregular shape, including a straight cylindrical bore having a circular cross section through the entire thickness of distal portion 16.

In another embodiment, as shown in Fig. 16H, exhalation port 22 includes two or more parts. The first is a cylindrical portion 128 and the second is a conical portion 130. Cylindrical portion 128 can be about 0.01 to 0.125 inches deep from back 27 of distal portion 16, with the remainder of exhalation port 22 being conical part 130. Conical portion 130 typically has an angle "β" which can be from about 1 degree to about 179 degrees, typically about 85 degrees.

Cylindrical portion 128 can have a diameter from about 0.005 inches to about 0.125 inches, preferably about 0.05 inches. In one embodiment, conical portion 130 can have a maximum diameter from about 0.01 inches to about 0.25 inches, preferably about 0.125 inches. In one embodiment, if holes or bores are used as exhalation ports 22, as shown in Fig. 16B, the center of the holes or bores can be positioned about 0.005 to 0.50 inches apart, preferably about 0.13 inches apart. The configuration shown, for example in 16B , i.e., the size and separate of exhalation ports 22, helps maintain the structural integrity of the device. In one embodiment, the shape of conical portion 130 can minimize the noise of air exiting the ports. In another embodiment, the conical portion can affect the leak rate of the present invention by maintaining an acceptable leak rate while simultaneously reducing noise.

In another embodiment, as shown in Figs. 6, 7, 10A, 10B, and 25A through 25E, an exhalation port can large enough to be fitted with nozzle 23. Nozzle 23 depicted in Figs. 6, 7, 10A, 10B and 25A through 25E can be made from hard plastic, such as polycarbonate, polyethylene, ceramic, acrylic, or any other material known in the art. In another embodiment, nozzle 23 can be made from a soft material, such as silicone.

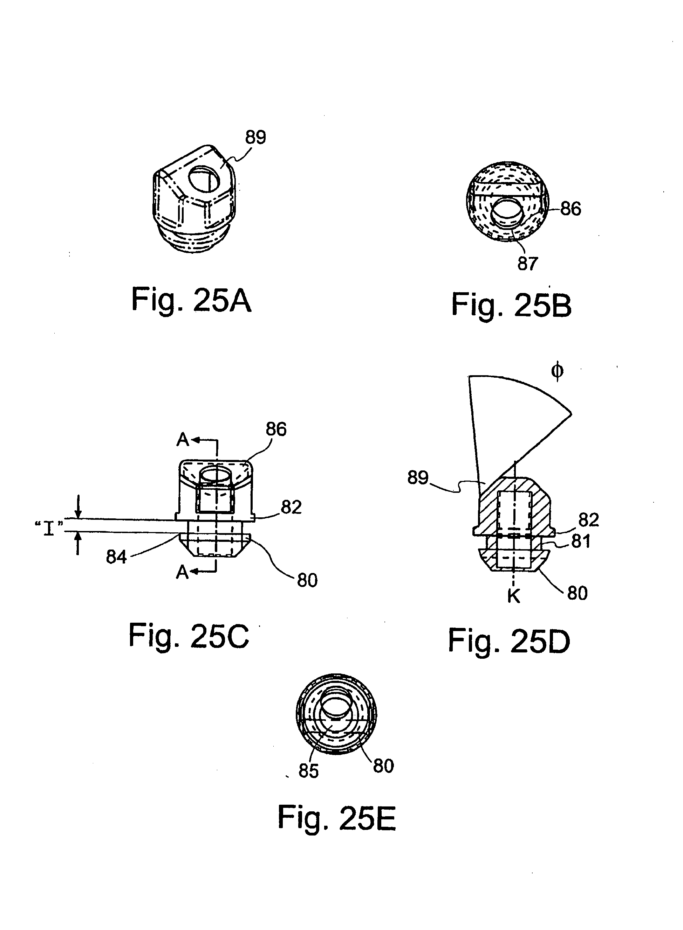

In particular, nozzle 23 as depicted in Figs. 25A through 25E can be inserted into exhalation port 22 of distal portion 16 of the present invention. For clarity, as shown in the Fig. 17B and illustrated generally by Fig. 10B, the exhalation ports 22 are also referenced as openings 68 and 70. Nozzle 23 can be held in place by inserting portion 80 into a hole 68 or 70 such that lips 82 and 84 engage, preferably sealingly engage, distal portion 16 at ridge 81.

In certain embodiments, the distance."I" between lips 82 and 84 can correspond to about the thickness of the distal portion of a nasal interface of the present invention where the exhalation port is inserted. In one embodiment, nozzle 23 depicted in Figs. 25A through 25E can be about 0.1 inches long to about 1.0 inches long, typically about 0.5 inches long.

Opening 85 of nozzle 23 depicted in Fig. 25E, on lower portion 80 can be any regular or irregular shape. In one embodiment, shown in Fig 25E, opening 85 is circular and is inserted into hole 68 of distal portion 16, as shown in Fig. 10B. Similarly, opening 87 on upper portion 86 of nozzle 23 can be any regular or irregular shape. In one embodiment, shown in Fig. 25B, opening 87 is circular. In one embodiment, opening 85 has a diameter of about 0.1 to 0.3 inches, typically about 0.2 inches and opening 87 can have a diameter of about 0.1 to about 0.3 inches, typically about 0.13 inches.

Because nozzle 23 depicted by Figs. 25A through 25E can be held in place by lips 82 and 84, nozzle 23 can swivel or be adjusted about the axis "K," shown in Fig. 25D. By incorporating an angled gas vent cut 89 into exhalation port 22, the direction of gas flow out of the device of the present invention can be controlled or directed by adjusting nozzle 23 about its axis. For example, as shown in Figs. 25A through 25F, opening 87 and opening 85 are at an angle "Φ" to one another, allowing for a user to direct exhaled CO2 in a desired direction by rotating nozzle 23 in distal portion 16.

With multiple sizes and interchangeable nasal prongs 10 and 12, a single nasal interface body will meet the needs of all patients. Accordingly, in one embodiment, the size of the exhalation ports 22 will remain the same for all patients. The advantage of consistent exhalation port sizes is that the interface of the present invention will function more consistently, thus likely providing more predictable ventilation therapy.

### E. Sealing Ring

In one embodiment, the present invention includes means for releasably, permanently engaging or locking proximal portion 14 to distal portion 16. Such means can include glue and adhesives, heating or melting the materials together, tabs, snaps, hooks and fasteners, and other fasteners such as those described herein that provides adequate sealing.

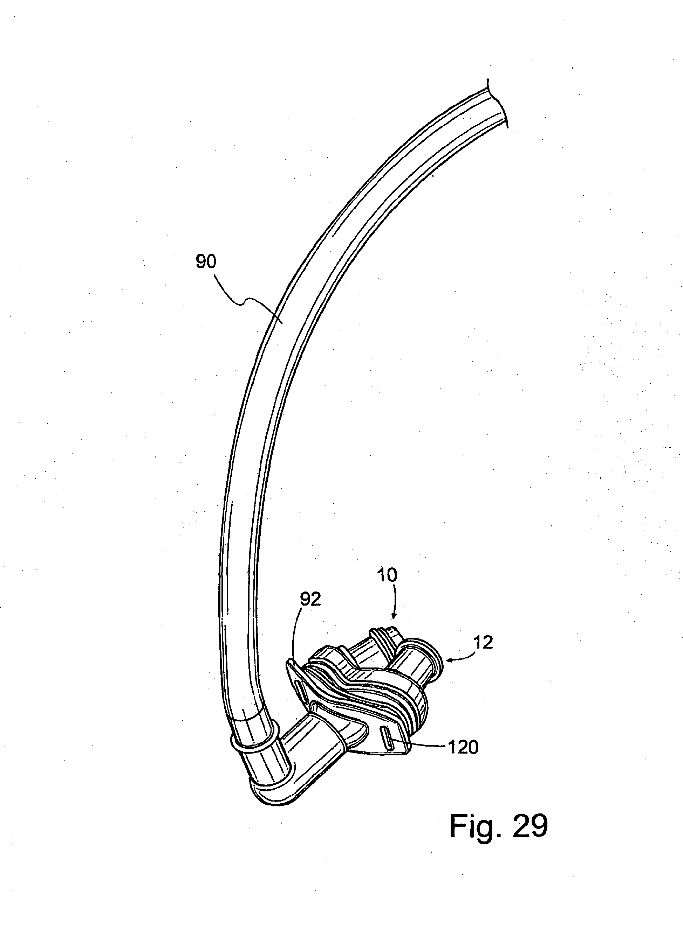

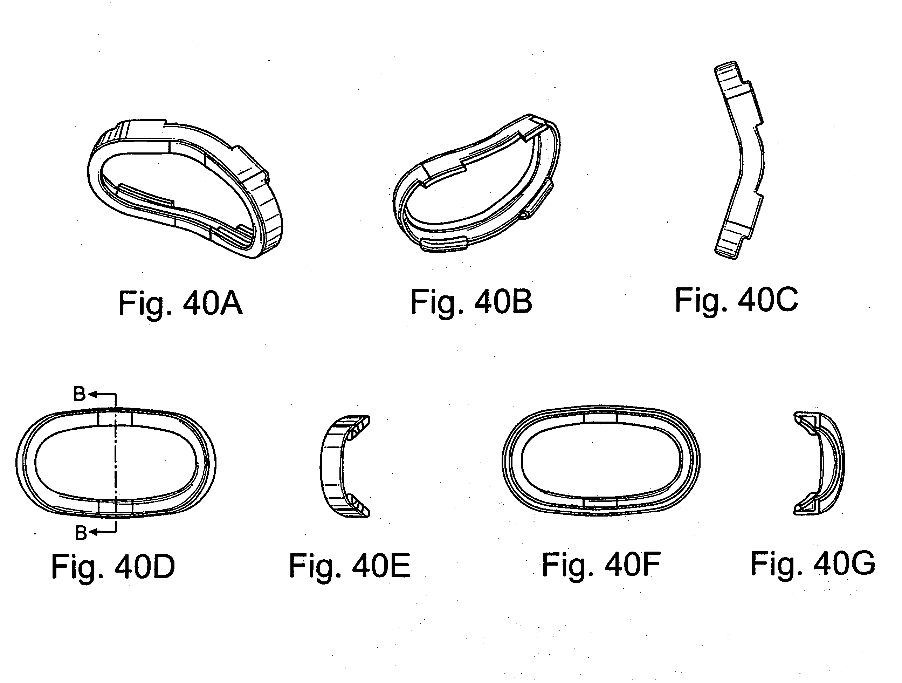

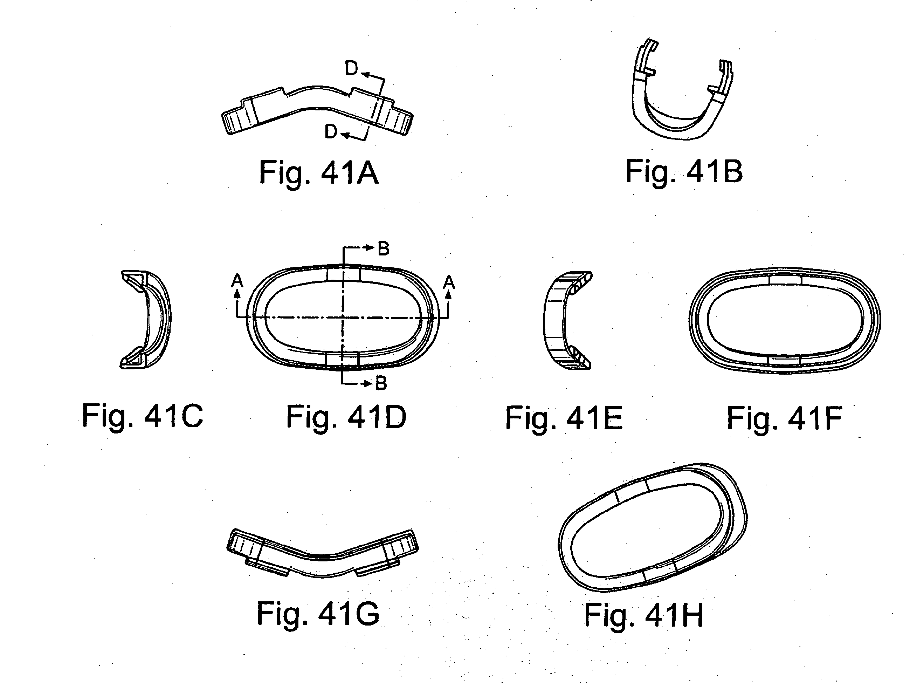

Embodiments of the present invention shown in Figs. 1 through 5, 9, 26, 27, 29, 30, and 35 through 38, include sealing ring 40. Sealing ring 40, also shown in Figs. 40A through 41H, can be any regular or irregular shape. In one embodiment, sealing ring 40 generally corresponds to the shape of proximal portion 14 and distal portion 16, as shown by the figures mentioned above. Sealing ring 40 can be made out of a hard, rigid, flexible or soft material. In some embodiments, sealing ring 40 can be made from hard plastic, such as polycarbonate, polyethylene, ceramic, acrylic, or any other material known in the art. In another embodiment, sealing ring 40 can be made from a soft material, such as silicone. In another embodiment, sealing ring 40 is made from the same material as proximal portion 14. In another embodiment, sealing ring 40 is made from the same material as distal portion 16. In some embodiments, material rigidity provides adequate force for sealing ring 40 to engage proximal portion 14 and distal portion 16 and retain each in place.

As noted above, sealing ring 40 facilitates the formation of a seal between proximal portion 14 and distal portion 16. Specifically, by stretching sealing ring 40 over proximal portion 14, sealing ring lip 43 can seat in groove 65, thereby forming a seal and removably securing or connecting sealing ring 40 to proximal portion 14. In another embodiment, proximal portion 14 can be inserted into sealing ring 40 such that sealing-ring lip 43 can seat in groove 65. Proximal portion 14 can then be sealingly secured to distal portion 16 by sealing tabs 42 which reversibly secure sealing ring 40, and thus the connected proximal portion 14, to distal portion 16 and can maintain extension 64 within lip 66, thereby maintaining a seal.

In one embodiment, sealing ring 40 is integral with or combined with proximal portion 14 to form a body of the invention. In another embodiment, proximal portion 14 is integral with or combined with distal portion 16 forming a body of the invention, without the need for sealing ring 40. In another embodiment, proximal portion 14, distal portion 16 and sealing ring 40 is integral with each other or combined together to form a body of the invention.

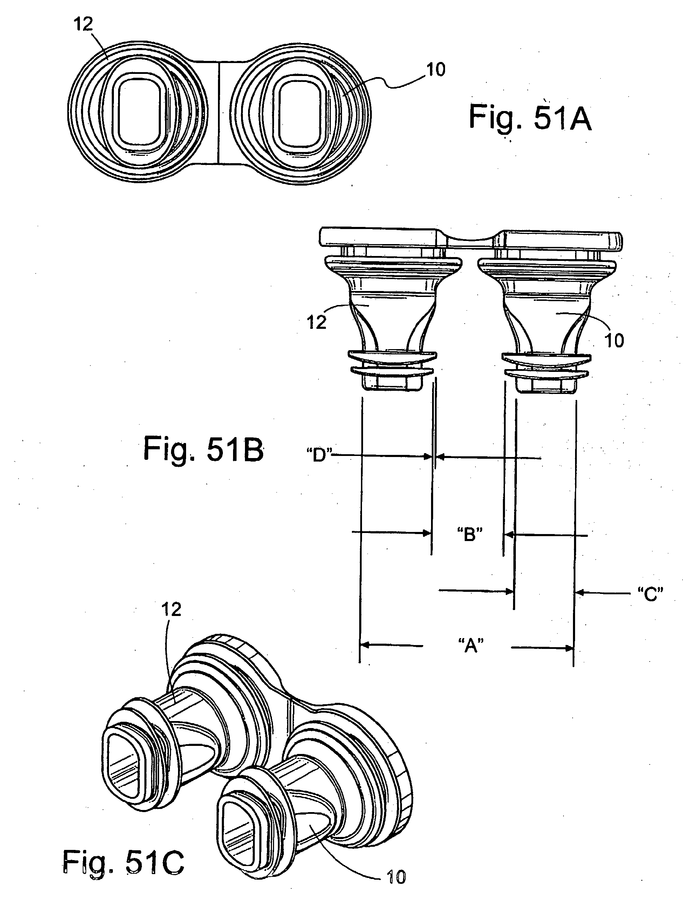

In certain embodiments where the distance between openings 60 and 62 of proximal portion 14 may change (i.e., the size of proximal portion 14 changes or the distances "A" or "B" of nasal prongs 10 and 12 change, as shown in Figs. 50 and 51), sealing ring 40 can be adapted to slidingly or movingly expand or contract to adjust to such changes. This embodiment provides the advantage of providing a single nasal interface that can accommodate multiple nasal prong sizes, and/or width of a patient's nares for a better fit..

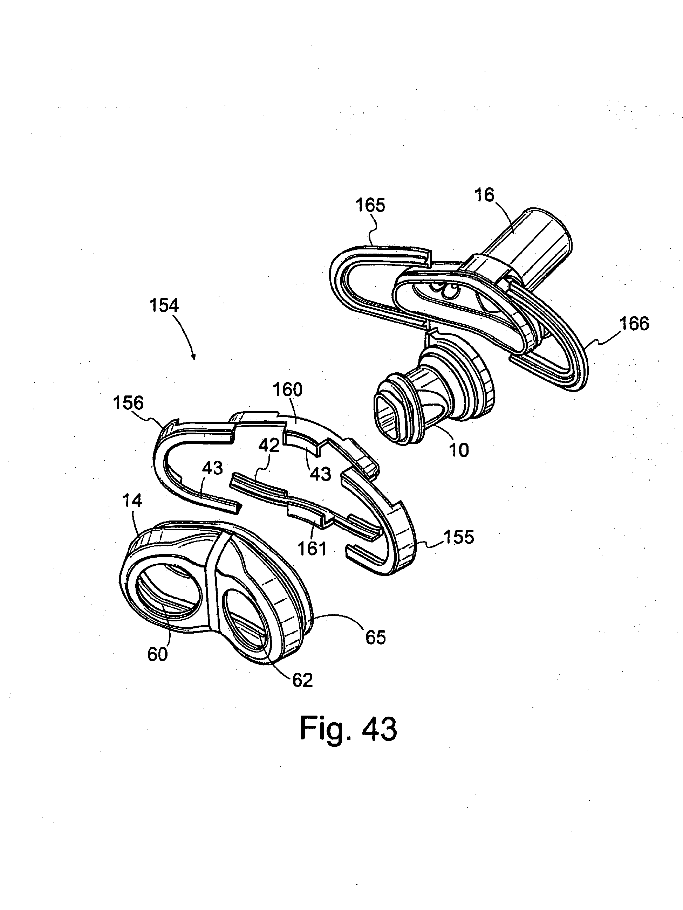

In one embodiment, as shown in Figs. 43 through 47, sealing lip 66 (e.g., as referred to in Fig. 16A through 16H) can be disengaged from distal portion 16, forming adjustable sealing lips 165 and 166 to thereby form a seal with proximal portion 14, as described above. To maintain the seal between proximal portion 14 and sealing lips 165 and 166 (and thus with distal portion 16), sealing ring 40 can be replaced with adjustable sealing ring 154. Adjustable sealing ring 154 can include sliding portions 155 and 156 and engaging portions 160 and 161.

Similar to the above described embodiments, sealing ring lip 43 of adjustable sealing ring 154 can seat in groove 65 of proximal portion 14, thereby forming a seal and removably securing or connecting adjustable sealing ring 154 to proximal portion 14. Proximal portion 14 can then be sealingly secured to distal portion 16 by sealing tabs 42 which reversibly secure adjustable sealing ring 154, and thus the connected proximal portion 14, to distal portion 16 by connecting to sealing lips 165 and 166, and can maintain extension 64 within sealing lip 66, thereby maintaining a seal.

In order to accommodate the needs of different patients, sliding portions 155 and 156 and can slidingly expand and contract depending upon the size of the nasal prongs 10 and/or 12 and/or the dimensions of the proximal portion 14 by sliding on engaging portions 161 and 162. In another embodiment utilizing adjustable sealing ring 154, as shown in Figs. 46 through 48, proximal portion 14 includes corrugation 150 (e.g., one or more folds in the material which permits expansion or contraction of the material), which, in one embodiment, permits the distance between openings 60 and 62 of proximal portion 14 to be manually adjusted depending upon the needs of the patient.

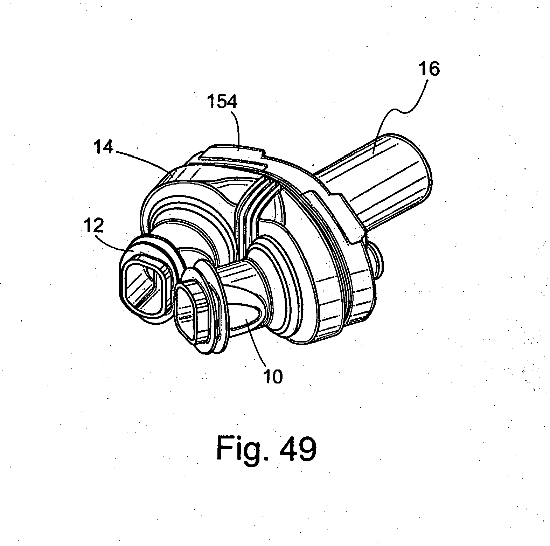

In one embodiment, a portion of adjustable sealing ring 154 releasably engages a portion of the distal portion 16 (e.g., sliding rails 167 and 168 and/or sealing lips 165 and 166) to connect the components of the invention together. As shown in Fig. 48, distal portion 16 further includes sliding rails 167 and 168. Sliding rails 167 and 168 permit sealing lips 165 and 166 to expand and contract and permit proximal portion 14, nasal prong 10 and/or 12, and sealing ring 154 to connect to distal portion 16. Preferably, the connections form at least substantially sealed connections. One embodiment of the invention of an assembled nasal interface including sliding rails 167 and 168 and sealing ring 154 is shown in Fig. 49.

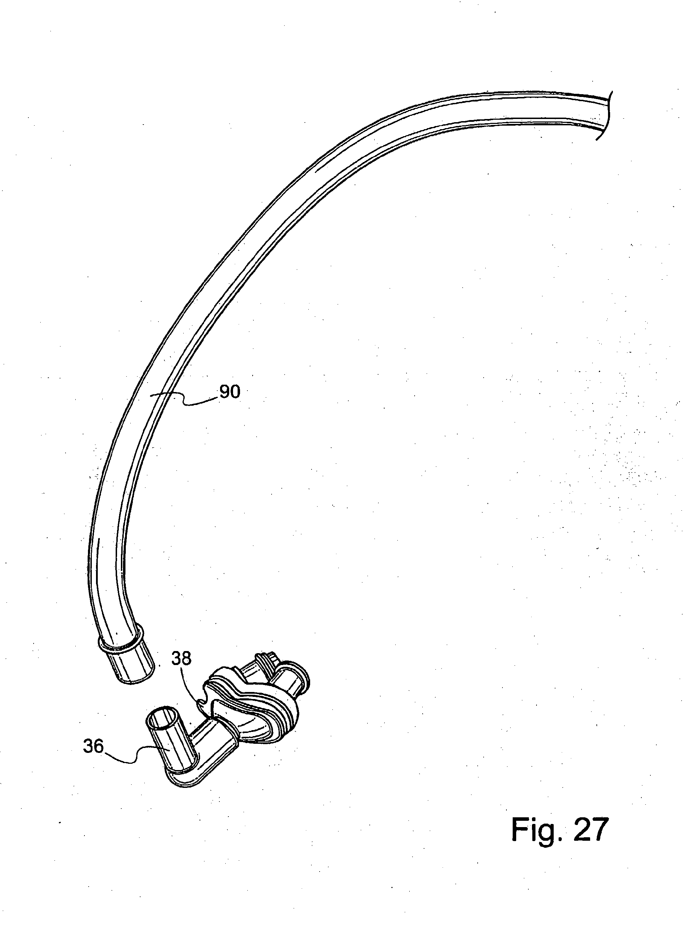

As described above, in one embodiment, proximal portion 14, is integral with or combined with nasal prongs 10 and 12. In yet another embodiment, sealing ring 40 is integral with or combined with proximal portion 14, which is also integral with or combined with nasal prongs 10 and 12, to form a body of the invention. In yet another embodiment, proximal portion 14, which is also integral with or combined with nasal prongs 10 and 12, is integral with or combined with distal portion 16 forming a body of the invention, without the need for sealing ring 40. In another embodiment, proximal portion 14, which is also integral with nasal prongs 10 and 12, distal portion 16 and sealing ring 40 can be integral with each other or combined together to form a body of the invention. For example, as shown in Fig. 27B, a device according to the present invention can be produced by integrally forming two or more components described herein.

Different components of the invention can be individually formed and/or formed as integral bodies, as described above by blow molding, injection molding, and/or overmolding. In embodiments where individual components (e.g., proximal portion 14 and distal portion 16) are made of different materials or materials having different durometers, overmolding is a preferred method of combining the separate components. Overmolding is a technique well known in the art and is described, e.g., in U.S. Patent No. 6,682,675, the entire content of which is hereby incorporated by reference..

In one embodiment, in order to facilitate the interchanging of nasal prongs, proximal portion 14 and/or distal portion 16 and/or sealing ring 40 can be connected to each other via one or more hinges, such that an embodiment of the invention can be opened at the hinge points to insert or interchange nasal prongs 10 and/or 12.

### III. Additional Components, System of the Invention and Methods of Use

The present invention further includes components that facilitate the use of the nasal interface of the present invention as a total treatment system. The additional components are described hereafter.

### A. Strap Connectors

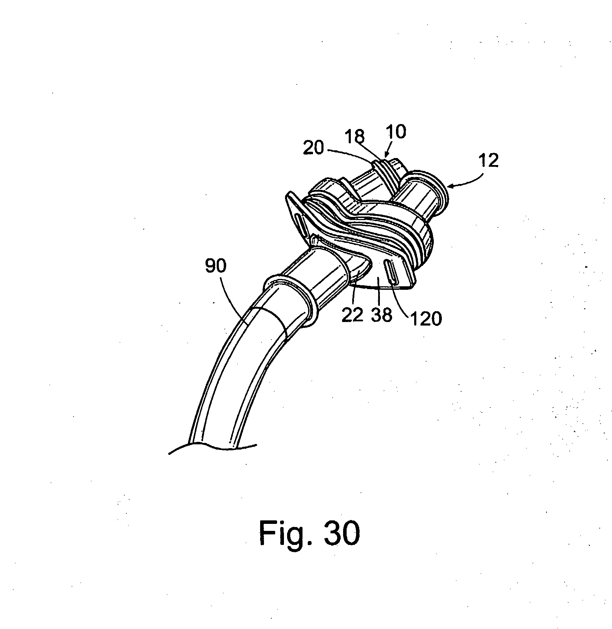

As described above, a nasal interface according to the present invention can also include one or more locking tabs 38 for releasably engaging a strap attachment plate 92, as shown in Fig. 30. One embodiment of a strap attachment plate 92 is shown in Figs. 28A through 28E.

Strap attachment plate 92 can be made out of a hard, rigid, flexible or soft material, and in some embodiments, strap attachment plate 92 can be made from fabric, neoprene, hard or soft plastic, such as polycarbonate, polyethylene, ceramic, acrylic, or any other material known in the art including metal. As shown in Fig. 28B, strap attachment plate 92 can be bent and includes one or more openings 136 for receiving one or more locking tabs 38, and includes one or more strap connections 120 for receiving a strap system 102, as detailed below.

Strap attachment plate 92 can facilitate the use of a strap system 102 by increasing the ease at which a strap can be connected to or removed from the nasal interface. Because the strap attachment plate is not integral to the nasal interface, the strap attachment plate, and any attached straps, can be more easily added or removed from the system.

Strap attachment plate 92 according to the invention can be from about 1.0 to 7.0 inches long, preferably about 3.5 inches long and can be about 0.1 to 3 inches wide, preferably about 1.2 inches wide.

In another embodiment, distal portion 16 also includes integral strap connections 28 and 30 (shown in Figs. 8 and Fig. 14C) so that a nasal interface according to the present invention can be held on the patient's face with a strap system 102, described below.

### B. Strap System

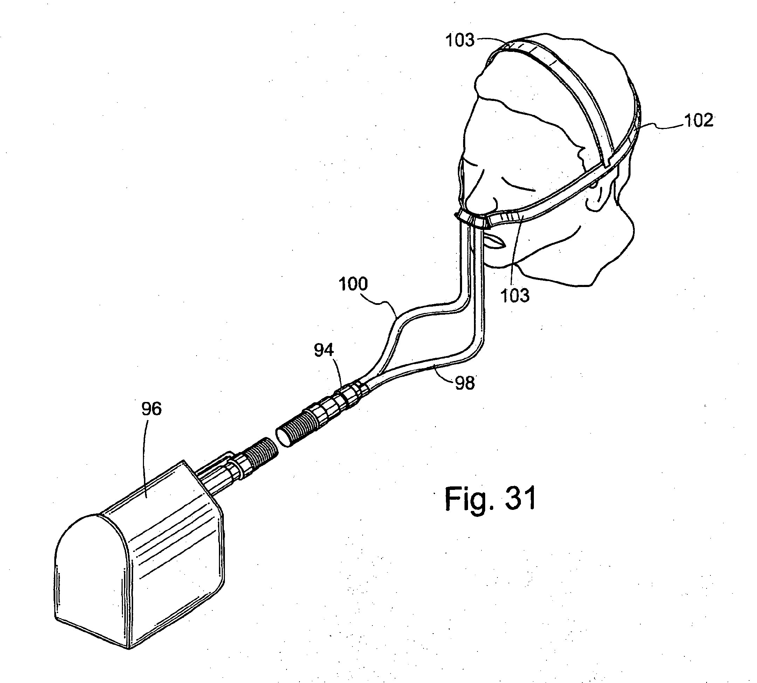

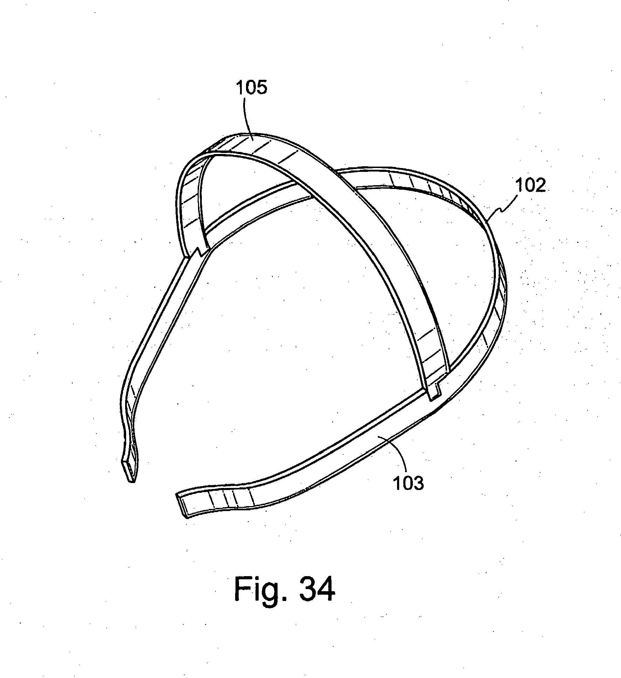

Preferably, strap system 102, shown in Figs. 31, 32, 34 through 38, and 42A through 42F, has a first strap 103 that attaches to the strap connections of the nasal interface of the present invention (e.g., strap connections 120, 28 or 30) at loop 122 and fits over a patient's head to surround the patient's head from the bottom of the nose to the back of the head. In one embodiment, first strap 103 can be about 33 inches long, but may be adjusted to fit heads of various sizes. For example, first strap 103 can have adjustable lengths of about 10 inches to about 70 inches.

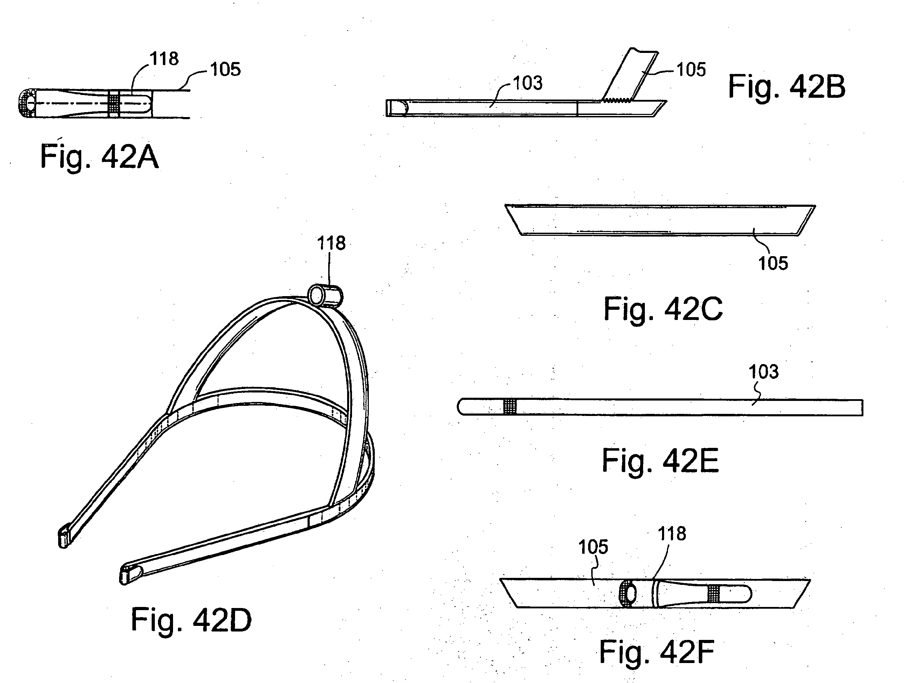

A second strap 105 can attach to first strap 103 and surrounds the top of the patient's head. In one embodiment, second strap 105 is about 22 inches long, but may also be adjusted to fit heads of various sizes. For example, second strap 105 may have adjustable lengths of about 5 inches to about 50 inches. Alternatively, strap system 102 may be available in different sizes for different sized heads. Strap system 102 may be made of any flexible or rigid material, but preferably is made of foam rubber with Velcro™ or another connective material ends to ease adjustability. In one embodiment, as shown in Figs. 42A, 42D and 42F, the present invention can include loop 118 for receiving tube 90, as shown in Fig. 37. A strap system can be fastened/unfastened using alternative methods to Velcro, such as snaps, buckles, buttons and ties.

It should be noted that in embodiments that are free of a strap systems, a nasal interface according to the present invention can be held onto the patient's face by nasal prongs 10 and 12 and nare sealing portions (e.g., flaps 18 and 20) and/or the pressure of the gas in nasal prongs 10 and 12 pressing against the nares.

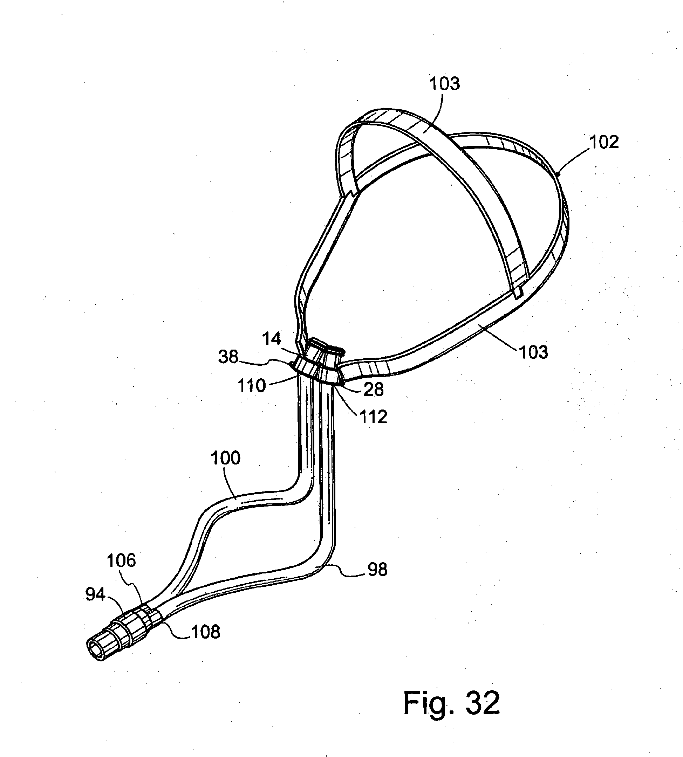

An assembled nasal interface and strap system 102 is shown in Fig. 32, where strap system 102 can be attached to strap connections 28 and 30. As shown in Figs. 31 and 32, in use, the patient will direct nasal prongs 10 and 12 into the nose and nasal prongs 10 and 12 can move into the nares until the cross section of the nare sealing portions (e.g., flaps 18 and 20) deforms or folds to match or approximate the cross section of each nare. The anatomy of the nose may deform the shape of nasal prongs 10 and 12 and their flaps 18 and 20 to achieve a comfortable seal. In one embodiment, the patient will also place strap system 102 over the head to secure the nasal interface to the patient's face.

### C. Tubing

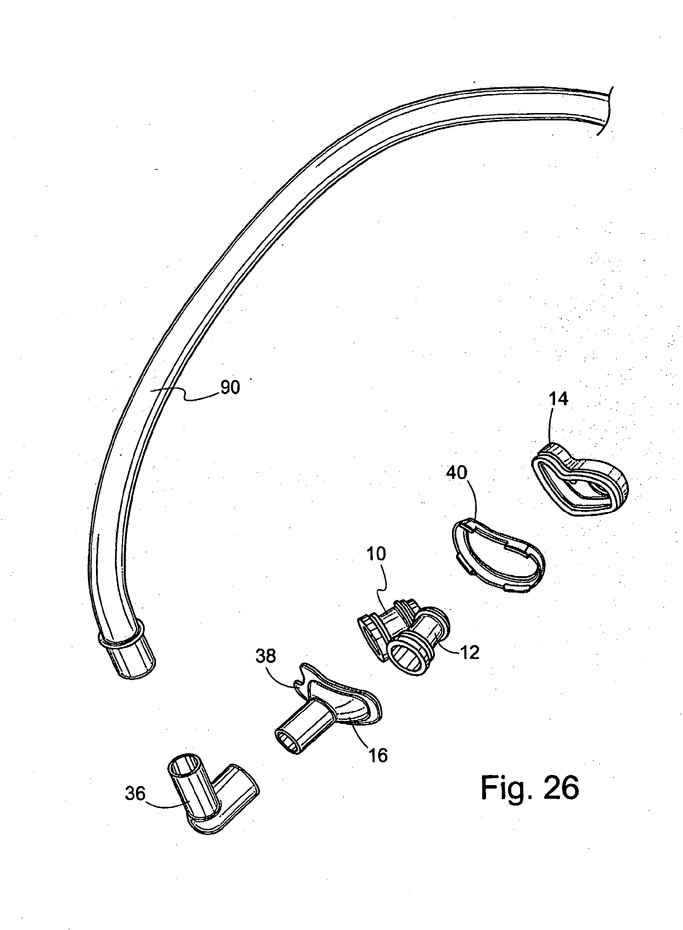

In one embodiment, the present invention includes tubing 90, shown in Figs. 26, 27, 29 and 35 through 38. Tubing 90 can supply gas to the nasal interface of the present invention. Tubing 90 can be made of any inert material, such as polyurethane, silicone, or another material known in the art.

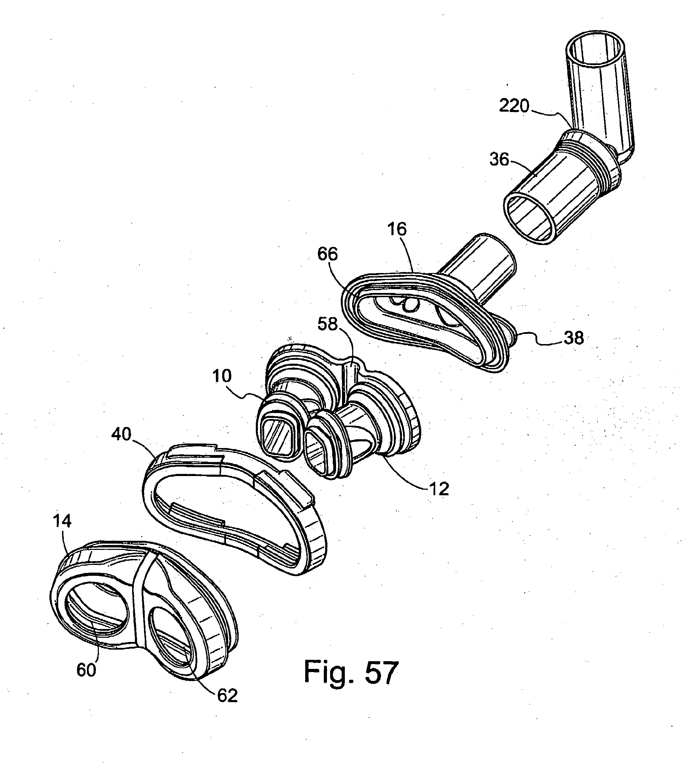

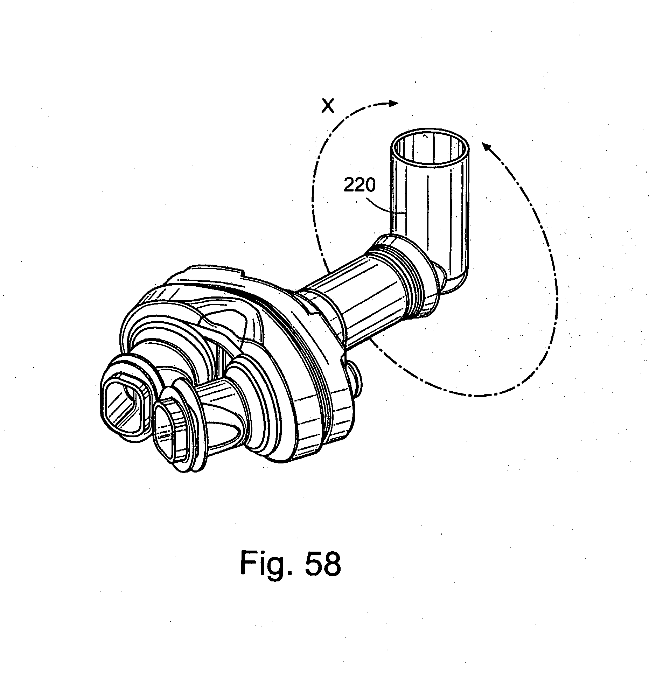

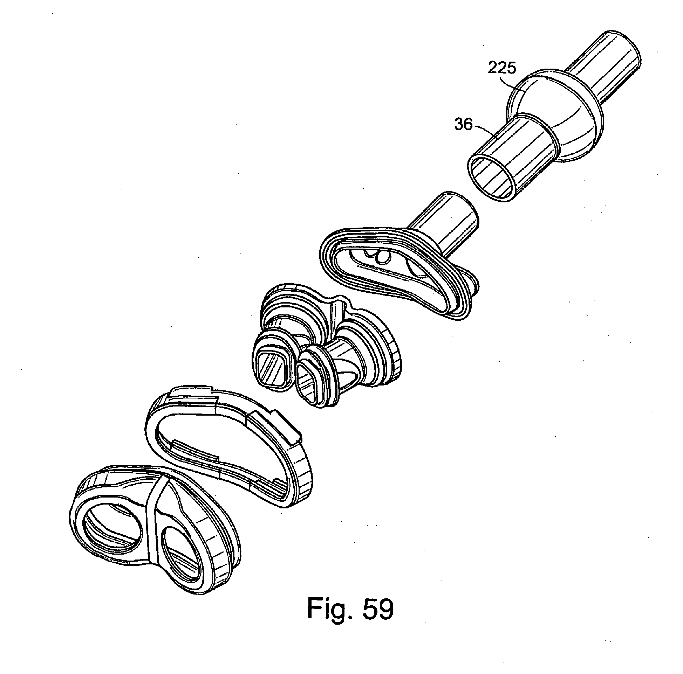

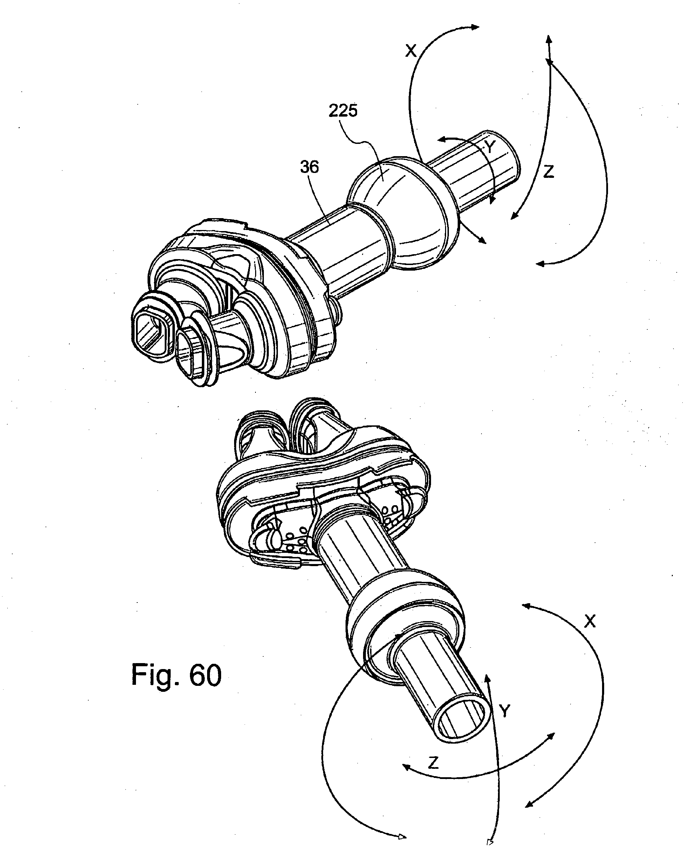

In another embodiment, as shown in Figs. 26, 27 and 29, the present invention includes tubing adapter 36. Tubing adapter 36 can be connected to distal portion 16 by inlet 24 and may have an angle "λ" as shown in Fig. 2. Angle "λ" may range from about 0° to 180°. In one embodiment, tubing adapter 36 can include an adjustable swivel 220 to permit adjustable movement of tubing adapter 36 from 0° through 360°, as shown in Figs 57 and 58. In another embodiment, the present invention can include ball joint 225 to permit free movement of tubing adapter 36 in multiple dimensions, including one or more of dimensions X, Y, and Z, as shown in Figs. 59 and 60. In one embodiment, swivel 220 and/or ball joint 225 form a seal to prevent or minimize gas leaks.

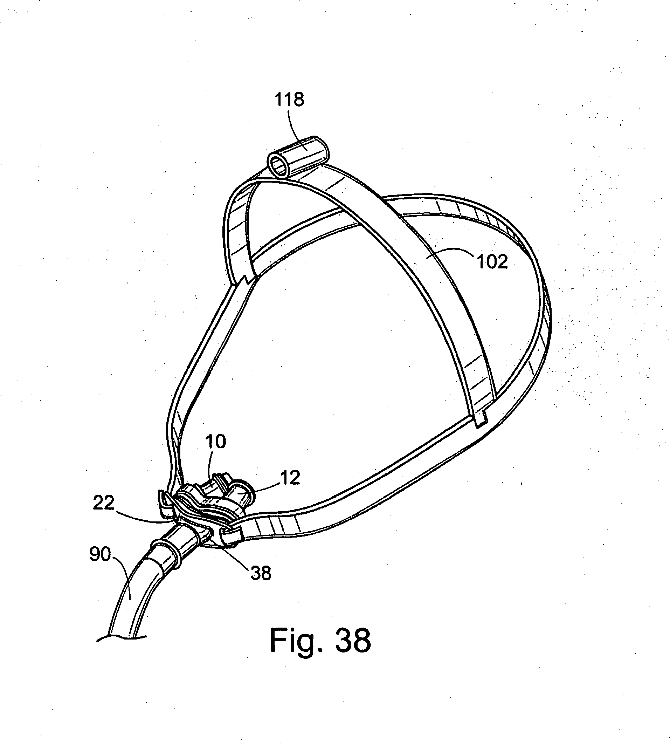

As shown in Fig. 30, one embodiment of the present invention includes tubing 90 directly connected to distal portion 16 and is free of tubing adapter 36. In one embodiment, the invention can include a single tubing 90 directed upward as shown in Fig. 37. In another embodiment, the invention can include a single tubing 90 directed downward, as shown in Fig. 38. In another embodiment, the invention can include a double tubing directed upward, in a manner similar to that shown in Fig. 37. In another embodiment, the invention can include a double tubing directed downward, as shown in Fig. 32.

With reference to Fig. 32, tubing 90 can connect to a tubing connector 94. Tubing connector 94 can operatively connect directly or indirectly with ventilation machine 96 (shown in Fig. 31) and is of a type known in the art for connecting tubing. In one embodiment, one single length of tubing connects to the nasal interface. In another embodiment, two lengths of tubing 98 and 100, each having a first end 106 and 108, respectively, and a second end 110 and 112, respectively, connect to the nasal interface, as shown in Fig. 32.

Second ends 110 and 112 fit over inlets 24 and 26 for connection to the nasal interface. In the two-inlet/two tubing system, tubings 98 and 100 may be of a smaller size than the tubing in the one-inlet/one tubing system because tubings 98 and 100 carry the same volume of gas as the single tube.

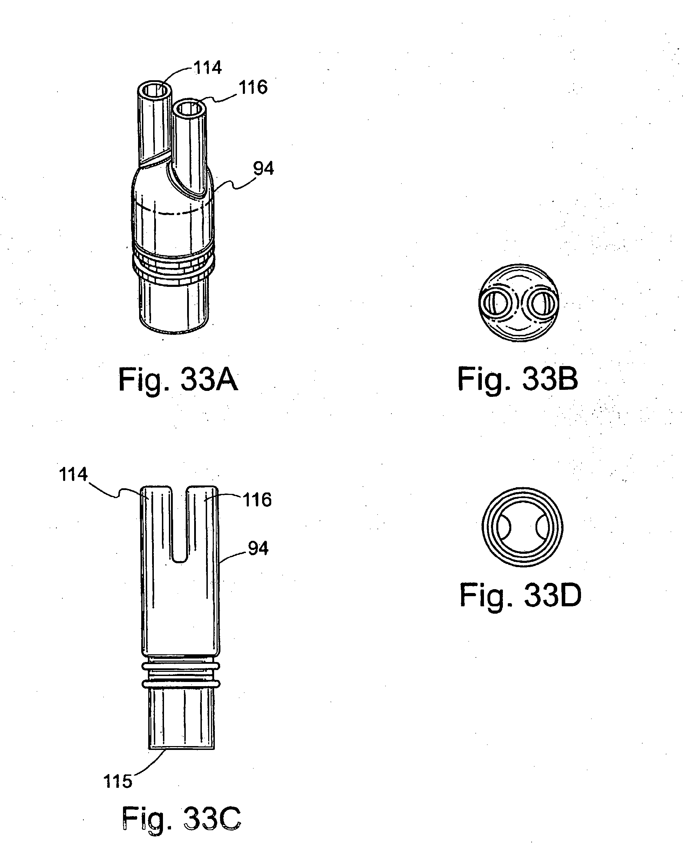

In one embodiment, tubing connector 94 is a three-way tubing junction removably attached to ends 106 and 108 of tubings 98 and 100. As shown in Figs. 33A through 33D, three-way tubing junction 94 may be provided by a "Y" junction, a "T" junction, or another junction as is known in the art and may or may not be a swivel connection. In one embodiment, connector 94 attaches directly or indirectly to ventilation machine 96 at an opposite end 115 from connectors 114 and 116.

A feed tube located on connector 94 at end 115, where such feed tube may be a separate component or attached to connector 94, is of a size selected to provide a sufficient air volume flow there through for full ventilation of the patient. For example, the size of the feed tube may be selected to accommodate about 120 liters of air per minute or about 5 liters of air per minute.

During operation of the ventilation system, gas is supplied through tubing 98 and 100. The gas may be air or oxygen-enriched air, or any gases or mists as may be desired in a given application. The gas is transported by the tubing to inlets 24 and 26, which direct the gas through the nasal interface and through nasal prongs 10 and 12 and into the nares of the patient. The nasal interface releases exhaled gases (e.g., CO₂) from exhalation ports 22 to the atmosphere.

In certain embodiments, it may be necessary to loop tubing 90. over strap 105. In such embodiments, strap 105 may further include a tube loop 118, which can retain the tube

In some embodiments, the system may also warm and humidify the delivered air. In other embodiments the system can deliver medications.

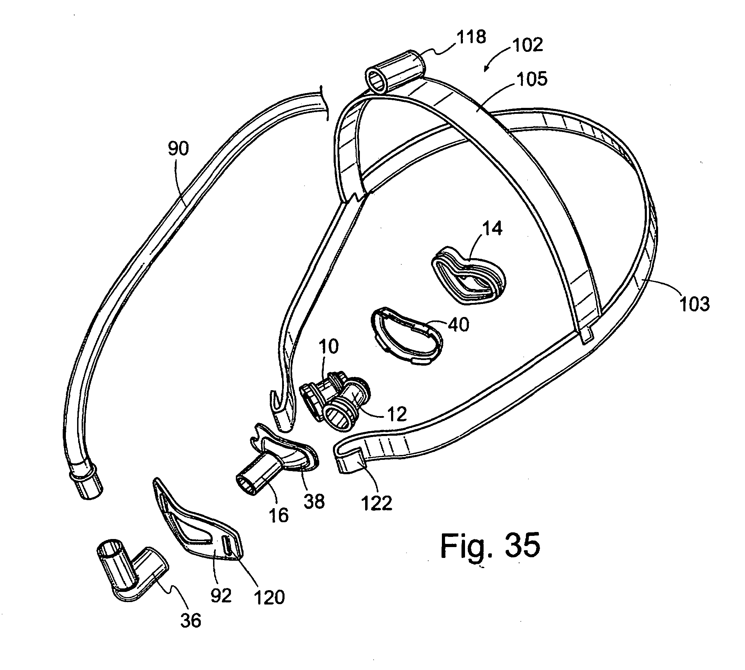

In other embodiments, shown in Figs. 35 through 38, a single tube 90 can be used to transport gas to single inlet 24 and into the nares of a nasal interface according to the present invention.

In light of the teachings above; one skilled in the art can also assemble a ventilation system of the present invention using alternative embodiments that include any one or more components or features of the above described embodiments. As shown in Figs. 35 through 38, ventilation systems can also be constructed using a strap system similar to that set forth above. Specifically, Fig. 35 shows an exploded view of one embodiment of the present invention and Fig. 37 shows an assembled view of the embodiment described in Fig. 35.

In one embodiment, shown in Figs. 39A and 39B, a nasal interface of the invention, including proximal portion 14, distal portion 16, nasal prongs 10 and 12, can bye connected to flexible tubing 90. Such an embodiment can have an overall length of about 5 to 50 inches, typically about 20 inches. Tubing 90 alone can have an overall length of about 5 to 50 inches, typically about 16 inches.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the spirit and scope of the appended claims.

## Claims

1. A nasal interface (2) for use in the nares of a patient for positive airway pressure applications comprising:
a pair of nasal prongs (10, 12), each prong having a bore, a first end, a second end;
a body having a distal portion (16) and a proximal portion (14) forming a chamber, the proximal portion (14) having apertures to receive the second ends of the nasal prongs (10, 12), the chamber being in communication with the bores of the nasal prongs;
at least one exhalation port (22) within the body;
at least one gas inlet (24) on the distal portion of the body, the at least one gas inlet (24) in communication with the chamber;
the nasal interface being **characterized in that** each prong (10, 12) comprises at least one deformable flap (18, 20) disposed proximate to the first end of each prong, for folding to match or approximate the cross-section of each nare, whereby a substantially airtight seal is created between the at least one deformable flap (18, 20) and an interior surface of the nares of the patient.

2. The device of claim 1, wherein the at least one flap (18, 20) retains the nasal interface (2) to the nares of a patient.

3. The device of claim 1, wherein the pair of nasal prongs (10, 12) are joined by a platform (58).

4. The device of claim 1, wherein the at least one flap (18, 20) includes a first flap (18) and a second flap (20) positioned proximate to the first flap (18), wherein a substantially airtight seal is created between the first and second flaps and an interior surface of the nares of the patient.

5. The device of claim 1, wherein the at least one flap (18, 20) has an oval shape.

6. The device of claim 5, wherein the second flap (20) extends radially outward more than the first flap (18) around its entire circumference.

7. The device of claim 1, wherein the pair of nasal prongs (10, 12) are sealingly engaged to the distal portion by a sealing ring.

8. The device of claim 1, wherein the pair of nasal prongs (10, 12) are adapted to releasably engage with the proximal portion of the body (14).

9. The device of claim 1, wherein the bore of each nasal prong (10, 12) at the first end has a first cross sectional shape and the bore at the second end has a second cross sectional shape.

10. The device of claim 1, wherein the nasal prongs (10, 12) include angled shoulder portions, the angled shoulder portions limiting movement of the nasal prongs into the nares of the patient.

11. The device of claim 1, wherein the distal portion (16) and the proximal portion (14) are releasably engaged and sealed by a sealing ring (40) so that the chamber is defined therebetween,

12. The device of claim 1, wherein the second ends of the nasal prongs are jointed by a platform (58), the platform being releasably engaged with the distal portion (16).

## Patentansprüche

1. Nasales Zwischenstück (2) zu Verwendung in den Nasenlöchern eines Patienten für positive Atemwegsdruckanwendungen, umfassend:
ein Paar von Nasenprongs (10, 12), wobei jeder Prong eine Bohrung, ein erstes Ende, ein zweites Ende aufweist;
einen Körper mit einem distalen Teil (16) und einem proximalen Teil (14), die eine Kammer bilden, wobei der proximale Teil (14) Öffnungen zur Aufnahme der zweiten Enden der Nasenprongs (10, 12) aufweist, wobei die Kammer in Kommunikation mit den Bohrungen der Nasenprongs steht;
wenigstens einen Exhalationsanschluss (22) in dem Körper;
wenigstens einen Gaseinlass (24) an dem distalen Teil des Körpers, wobei sich der wenigstens eine Gaseinlass (24) in Kommunikation mit der Kammer befindet;
wobei das nasale Zwischenstück darin gekennzeichnet ist, dass jeder Prong (10, 12) wenigstens eine verformbare Klappe (18, 20) umfasst, die proximal zu dem ersten Ende jedes Prongs angeordnet ist, um so geklappt zu werden, dass eine Angleichung oder Annäherung an den Querschnitt jedes Nasenloch erfolgt, wodurch ein im Wesentlichen luftdichter Verschluss zwischen der wenigstens einen verformbaren Klappe (18, 20) und einer inneren Oberfläche der Nasenlöcher des Patienten erzeugt wird.

2. Vorrichtung nach Anspruch 1, wobei die wenigstens eine Klappe (18, 20) das nasale Zwischenstück (2) an den Nasenlöchern eines Patienten hält.

3. Vorrichtung nach Anspruch 1, wobei das Paar von Nasenprongs (10, 12) durch eine Plattform (58) verbunden ist.

4. Vorrichtung nach Anspruch 1, wobei die wenigstens eine Klappe (18, 20) eine erste Klappe (18) und eine zweite Klappe (20) aufweist, die proximal zu der ersten Klappe (18) positioniert ist, wobei ein im Wesentlichen luftdichter Verschluss zwischen den ersten und zweiten Klappen und einer inneren Oberfläche der Nasenlöcher des Patienten erzeugt wird.

5. Vorrichtung nach Anspruch 1, wobei die wenigstens eine Klappe (18, 20) eine ovale Form aufweist.

6. Vorrichtung nach Anspruch 5, wobei sich die zweite Klappe (20) radial auswärts mehr erstreckt als die erste Klappe (18) um ihren gesamten Umfang.

7. Vorrichtung nach Anspruch 1, wobei das Paar von Nasenprongs (10, 12) durch einen Dichtungsring abdichtend mit dem distalen Teil eingreift.

8. Vorrichtung nach Anspruch 1, wobei das Paar von Nasenprongs (10, 12) lösbar mit dem proximalen Teil des Körpers (14) eingreifen kann.

9. Vorrichtung nach Anspruch 1, wobei die Bohrung jedes Nasenprongs (10, 12) an dem ersten Ende eine erste Querschnittsform aufweist, und wobei die Bohrung an dem zweiten Ende eine zweite Querschnittsform aufweist.

10. Vorrichtung nach Anspruch 1, wobei die Nasenprongs (10, 12) angewinkelte Schulterabschnitte aufweisen, wobei die angewinlcelten Schulterabschnitte die Bewegung der Nasenprongs in die Nasenlöcher des Patienten begrenzen.

11. Vorrichtung nach Anspruch 1, wobei der distale Teil (16) und der proximale Teil (14) lösbar miteinander eingreifen und durch einen Dichtungsring (40) abgedichtet sind, so dass die Kammer dazwischen definiert wird.

12. Vorrichtung nach Anspruch 1, wobei die zweiten Enden der Nasenprongs durch eine Plattform (58) verbunden sind, wobei die Plattform lösbar mit dem distalen Teil (16) eingreift.

## Revendications

1. Interface nasale (2) destinée à être utilisée dans les narines d'un patient pour des applications de pression positive dans les voies respiratoires comprenant :
une paire de canules nasales (10, 12), chaque canule ayant un alésage, une première extrémité, une seconde extrémité ;
un corps ayant une partie distale (15) et une partie proximale (14) formant une chambre, la partie proximale (14) ayant des ouvertures pour recevoir les secondes extrémités des canules nasales (10, 12), la chambre étant en communication avec les alésages des canules nasales ;
au moins un orifice d'expiration (22) dans le corps ;
au moins une entrée de gaz (24) sur la partie distale du corps, l'au moins une entrée de gaz (24) étant en communication avec la chambre ;
l'interface nasale étant **caractérisée en ce que** chaque canule (10, 12) comprend au moins un rabat déformable (18, 20) disposé à proximité de la première extrémité de chaque canule, afin d'être plié pour correspondre totalernent ou partiellement à la section transversale de chaque narine, un joint sensiblement étanche à l'air étant ainsi créé entre l'au moins un rabat déformable (18, 20) et une surface interne des narines du patient.

2. Dispositif selon la revendication 1, l'au moins un rabat (18, 20) retenant l'interface nasale (2) sur les narines d'un patient.

3. Dispositif selon la revendication 1, la paire de canules nasales (10, 12) étant jointe par une plate-forme (58).

4. Dispositif selon la revendication 1, l'au moins un rabat (18, 20) comprenant un premier rabat (18) et un second rabat (20) positionné à proximité du premier rabat (18), un joint sensiblement étanche à l'air étant créé entre les premier et second rabats et une surface interne des narines du patient.

5. Dispositif selon la revendication 1, l'au moins un rabat (18, 20) ayant une forme ovale.

6. Dispositif selon la revendication 5, le second rabat (20) s'étendant radialement vers l'extérieur plus que le premier rabat (18) sur toute sa circonférence.

7. Dispositif selon la revendication 1, la paire de canules nasales (10, 12) entrant en contact étanche avec la partie distale par un anneau d'étanchéité.

8. Dispositif selon la revendication 1, la paire de canules nasales (10, 12) étant conçue pour entrer en contact amovible avec la partie proximale du corps (14).

9. Dispositif selon la revendication 1, l'alésage de chaque canule nasale (10, 12) au niveau de la première extrémité ayant une première forme en section transversale et l'alésage au niveau de la seconde extrémité ayant une seconde forme en section transversale.

10. Dispositif selon la revendication 1, les canules nasales (10, 12) comprenant des parties d'épaulement inclinées, les parties d'épaulement inclinées limitant le mouvement des canules nasales dans les narines du patient.

11. Dispositif selon la revendication 1, la partie distale (16) et la partie proximale (14) entrant en contact amovible et étant rendues étanches par un anneau d'étanchéité (40) de sorte que la chambre soit définie entre elles.

12. Dispositif selon la revendication 1, les secondes extrémités des canules nasales étant jointes par une plate-forme (58), la plate-forme entrant en contact amovible avec la partie distale (16).
